# EUROPÄISCHE PATENTSCHRIFT

(11) **EP 3 824 836 B1**
(45) Veröffentlichungstag und Bekanntmachung des Hinweises auf die Patenterteilung: **12.02.2025**
(21) Anmeldenummer: 19210911.4
(22) Anmeldetag: 22.11.2019
(51) Int. Cl.: A61B 18/14

(54) **SONDE**
PROBE
SONDE

(43) Veröffentlichungstag der Anmeldung: 26.05.2021
(73) Patentinhaber: Erbe Elektromedizin GmbH, 72072 Tübingen (DE)
(72) Erfinder: Artes, Charlotte, 72411 Bodelshausen (DE); Wandel, Waldemar, 72127 Kusterdingen (DE)
(74) Vertreter: Rüger Abel Patentanwälte PartGmbB

(56) Entgegenhaltungen:
- CN-A- 101 579 256
- US-A- 6 033 404
- US-A1- 2007 149 968
- US-A1- 2015 088 130
- US-A1- 2019 328 451
- US-B2- 10 080 605
- US-B2- 8 814 856
- US-B2- 9 138 251
- US-B2- 9 622 768

## Beschreibung

Die Erfindung betrifft eine Sonde zur elektrochirurgischen Behandlung von Gewebe. Die Sonde hat einen Sondenkörper mit einer darin bewegbar angeordneten Elektrode. Bei der Sonde kann es sich um eine monopolare Sonde mit einer einzigen Elektrode oder bipolare Sonde mit mehreren Elektroden handeln. Mittels einer Bedienvorrichtung kann die Elektrode aus dem Sondenkörper ausgefahren bzw. in den Sondenkörper eingefahren werden. In ausgefahrenem Zustand kann an die Elektrode eine Spannung angelegt werden, insbesondere um Gewebe zu schneiden.

Solche Sonden sind aus der Praxis bekannt. Für einen Operateur besteht der Bedarf, die aus dem Sondenkörper ausgefahrene Länge des distalen Endabschnitts der Elektrode exakt einstellen zu können. Dadurch kann verhindert werden, dass die Elektrode beim Schneiden von Gewebe in tiefere Gewebeschichten eindringt, die während des Eingriffs nicht geschnitten bzw. beschädigt werden sollen.

US 8 814 856 B2 beschreibt eine Sonde mit einer Bedienvorrichtung, mittels der eine Elektrode aus einem Schaft ausgefahren werden kann. Die Bewegungskopplung zwischen der Elektrode und der Bedienvorrichtung ist durch ein Untersetzungsgetriebe erreicht, das als Hebelgetriebe ausgebildet ist.

US 2019/0328451 A1 beschreibt eine Sonde gemäß dem Oberbegriff des Patentanspruches 1, die als kombinierte Sonde ausgebildet ist. Die Sonde hat zwei koaxial zueinander angeordnete Elemente, die rohrförmig gestaltet sind. Durch das innere Element kann ein Fluid gefördert und am distalen Ende abgegeben werden. Das äußere Element kann beispielsweise als Elektrode dienen. Die beiden Elemente können separat individuell gegenüber einem gemeinsamen Körper und relativ zueinander bewegt werden. Hierzu ist eine Bedienvorrichtung vorhanden. US 2007/149968 A1 und US 2015/088130 A1 beschreiben auch kombinierte Sonden.

Somit ist es die Aufgabe der vorliegenden Erfindung, eine Sonde zu schaffen, die eine verbesserte Handhabung für einen Operateur ermöglicht.

Diese Aufgabe wird mit einer Sonde gemäß Patentanspruch 1 gelöst.

Die erfindungsgemäße Sonde ist zur elektrochirurgischen Behandlung von Gewebe eingerichtet. Sie weist einen Sondenkörper auf, in dem sich ein Elektrodenkanal bis zu einem distalen Ende des Sondenkörpers erstreckt. Am proximalen Ende ist der Sondenkörper mit einer Bedienvorrichtung verbunden. Der Sondenkörper kann als starres Rohr und vorzugsweise als flexibler Schlauch ausgebildet sein. Unter einem starren Rohr ist ein Sondenkörper zu verstehen, der sich bei den während der Benutzung der Sonde üblicherweise auftretenden Kräften nicht biegen lässt. Demgegenüber ist unter einem flexiblen Schlauch ein Sondenkörper zu verstehen, der bei den üblicherweise auftretenden Kräften während des bestimmungsgemäßen Gebrauchs der Sonde gegenüber einer ursprünglichen Erstreckungsrichtung gebogen werden kann. Eine Sonde mit einem flexiblen Schlauch als Sondenkörper kann beispielsweise durch einen Endoskopkanal eines Endoskops geführt werden.

Die Bedienvorrichtung hat ein Gehäuse. An dem Gehäuse ist ein Elektroden-Bedienelement angeordnet. Das Elektroden-Bedienelement ist zwischen einer ersten Stellung und einer zweiten Stellung bewegbar, beispielsweise linear verschiebbar. Alternativ kann das Elektroden-Bedienelement zwischen der ersten Stellung oder der zweiten Stellung auch schwenkbar am Gehäuse angeordnet sein.

In dem Elektrodenkanal des Sondenkörpers ist eine Elektrode angeordnet, die entlang der Erstreckungsrichtung des Elektrodenkanals bewegbar gelagert ist. Das distale Ende der Elektrode kann zwischen einer vollständig ausgefahrenen Stellung und einer vollständig eingefahrenen Stellung bewegt werden. In der vollständig ausgefahrenen Stellung ist ein sich an das distale Ende der Elektrode anschließender distaler Endabschnitt, der sich außerhalb des Elektrodenkanals befindet, am längsten. In der vollständig eingefahrenen Stellung befindet sich das distale Ende der Elektrode innerhalb des Elektrodenkanals.

Zum Einfahren und Ausfahren der Elektrode dient das Elektroden-Bedienelement der Bedienvorrichtung. Das Elektroden-Bedienelement ist vorzugsweise mittels eines Untersetzungsgetriebes mit der Elektrode bewegungsgekoppelt. Das Untersetzungsgetriebe ist dazu eingerichtet, eine Bewegung des Elektroden-Bedienelements in eine entsprechend des Untersetzungsverhältnisses kürzere Bewegung der Elektrode zu untersetzen. Das heißt, dass ein Bedienweg des Elektroden-Bedienelements größer ist als der Weg, den die Elektrode zurücklegt, wenn das Elektroden-Bedienelement entlang des Bedienwegs bewegt wird. Insbesondere ist der maximal zur Verfügung stehende Bedienweg zwischen der ersten Stellung und der zweiten Stellung größer als der Weg der Elektrode zwischen der vollständig ausgefahrenen Stellung und der vollständig eingefahrenen Stellung. Die Untersetzung des Untersetzungsgetriebes ist bevorzugt konstant und nicht stellungs- oder wegabhängig.

Aufgrund der Untersetzung kann der Operateur mittels eines langen Bedienwegs des Elektroden-Bedienelements eine sehr genaue Positionierung der Elektrode erreichen. Dadurch lässt sich das distale Ende der Elektrode einfach und genau positionieren, insbesondere mit einer Genauigkeit im Sub-Millimeterbereich.

Es ist vorteilhaft, wenn das Untersetzungsgetriebe ein Hebelgetriebe ist. Bei einem Ausführungsbeispiel kann das Hebelgetriebe einen an einer Schwenklagerstelle am Gehäuse der Bedienvorrichtung gelagerten Hebel aufweisen. Vorzugsweise hat das Hebelgetriebe nur einen einzigen Hebel. Eine Kopplungsstelle, an der das Elektroden-Bedienelement mit dem Hebel gekoppelt ist, ist weiter von der Schwenklagerstelle entfernt, als eine Kopplungsstelle, an der der Hebel mit der Elektrode gekoppelt ist. Auf diese Weise wird ein einfach ausgestaltetes Hebelgetriebe mit einer Untersetzung erreicht.

Die Schwenklagerstelle kann an einem Ende des Hebels angeordnet sein.

Bei einer Ausführungsform befindet sich die Kopplungsstelle der Elektrode mit dem Hebel zwischen der Schwenklagerstelle und der Kopplungsstelle, an der das Elektroden-Bedienelement am Hebel angreift.

Anstelle eines Hebelgetriebes können auch andere Getriebearten verwendet werden, wie etwa ein Exzentergetriebe, ein Getriebe mit einer Zahnstange und einem Zahnrad, ein Zahnradgetriebe, ein Riemengetriebe, ein Reibradgetriebe usw. Auch Kombinationen der beschriebenen Getriebearten sind möglich. Das Untersetzungsgetriebe kann eine oder mehrere Getriebestufen aufweisen.

Bei einer weiteren vorteilhaften Ausgestaltung, die insbesondere auch unabhängig vom Untersetzungsgetriebe realisiert werden kann, weist die Sonde eine Rasteinrichtung auf. Die Rasteinrichtung kann mehrere unterschiedliche Positionen der Elektrode relativ zum Sondenkörper definieren. Insbesondere kann die Rasteinrichtung diese Positionen durch jeweils eine Raststellung definieren. Durch die Raststellungen der Rasteinrichtung lassen sich definierte, vorgegebene Positionen der Elektrode und insbesondere der Abstand des distalen Endes der Elektrode vom distalen Ende des Sondenkörpers exakt einstellen, so dass dem Operateur das Positionieren der Elektrode erleichtert wird.

Bei einem Ausführungsbeispiel definiert die Rasteinrichtung eine vollständig ausgefahrene Position der Elektrode, eine vollständig eingefahrene Position der Elektrode und wenigstens eine Zwischenposition der Elektrode zwischen der vollständig ausgefahrenen Position und der vollständig eingefahrenen Position.

Die Rasteinrichtung hat bei einer bevorzugten Ausführungsform ein mit dem Elektroden-Bedienelement bewegungsverbundenes Rastelement und ein mit dem Gehäuse der Bedienvorrichtung bewegungsverbundenes Rastgegenelement. Vorzugsweise ist das Rastelement gegenüber dem Elektroden-Bedienelement unbeweglich angeordnet. Vorzugsweise ist das Rastgegenelement gegenüber dem Gehäuse unbeweglich angeordnet. Das Rastelement und das Rastgegenelement stellen in jeder vorgegebenen definierten Position der Elektrode gegenüber dem Sondenkörper eine Raststellung ein, in der eine lösbare Rastverbindung zwischen dem Rastelement und dem Rastgegenelement hergestellt ist.

Das Rastelement kann wenigstens einen Rastvorsprung und/oder wenigstens eine Rastausnehmung aufweisen. Das Rastgegenelement kann zur Zusammenarbeit mit dem Rastelement wenigstens eine Rastausnehmung und/oder wenigstens einen Rastvorsprung aufweisen. Beispielsweise kann ein einziger Rastvorsprung vorhanden sein, der in unterschiedlichen definierten Positionen der Elektrode in jeweils eine zugeordnete Rastausnehmung eingreift. Umgekehrt kann eine einzige Rastausnehmung vorhanden sein, in die jeweils eines von mehreren Rastelementen in den unterschiedlichen definierten Positionen der Elektrode eingreift. In jeder Raststellung - bei hergestellter lösbarer Rastverbindung - können auch mehrere Rastvorsprünge in mehrere zugeordnete Rastausnehmungen eingreifen.

Bei allen erläuterten Ausgestaltungen kann der Rastvorsprung beispielsweise elastisch bewegbar gelagert sein, so dass der Rastvorsprung eine Relativbewegung des Elektroden-Bedienelements gegenüber dem Gehäuse der Bedienvorrichtung aus einer Raststellung heraus ermöglicht. Beispielsweise kann eine elastisch oder federelastisch vorgespannte Kugel oder ein anderer Körper einen Rastvorsprung bilden, die aufgrund der Federverspannung in eine Vertiefung oder ein Loch eingreift, das eine Rastausnehmung darstellt. Der Rastvorsprung kann durch eine Feder oder auch durch ein elastisch verformbares Element vorgespannt und in und entgegen dieser Vorspannkraft beweglich gelagert sein, beispielsweise durch eine Art Filmscharnier.

Bei einer bevorzugten Ausführungsform weist die Sonde eine Vorspanneinrichtung auf. Die Vorspanneinrichtung beaufschlagt die Elektrode gegenüber dem Sondenkörper in der Erstreckungsrichtung der Elektrode bzw. des Elektrodenkanals mit einer Vorspannkraft. Die Vorspanneinrichtung kann eine oder mehrere Vorspannelemente aufweisen, insbesondere wenigstens eine Feder und vorzugsweise Schraubenfeder. Durch die Vorspannkraft kann ein Bewegungsspiel der Elektrode in Erstreckungsrichtung eliminiert werden.

Es ist bevorzugt, wenn die Vorspannkraft der Vorspanneinrichtung die Elektrode in Richtung der vollständig ausgefahrenen Stellung drängt.

Es ist insbesondere auch vorteilhaft, wenn die Vorspannkraft einen Betrag aufweist, der größer ist als die bei der Verwendung der Sonde auf die Elektrode einwirkende Gegenkraft beim Schneiden von Gewebe.

Bei einer bevorzugten Ausführungsform ist die Vorspanneinrichtung oder zumindest ein Vorspannelement der Vorspanneinrichtung im Sondenkörper bzw. im Elektrodenkanal angeordnet und kann sich beispielsweise einerseits am Sondenkörper und andererseits an der Elektrode abstützen. Bevorzugt ist die Vorspanneinrichtung oder zumindest ein Vorspannelement distal angeordnet, insbesondere entweder direkt hinter einem distalen Endstück des Sondenkörpers oder mit einen Abstand von beispielsweise 10-15cm vom Endstück entfernt, also sozusagen nach proximal zurückversetzt. Durch einen solchen Abstand kann ein Klemmen der Vorspanneinrichtung oder des zumindest einen Vorspannelements beim Biegen des Sondenkörpers bzw. eines Endoskops im distalen Endbereich vermieden werden.

Alternativ oder zusätzlich kann die Vorspanneinrichtung oder zumindest ein Vorspannelement der Vorspanneinrichtung im Gehäuse der Bedienvorrichtung angeordnet sein.

Die Sonde ist erfindungsgemäß als kombinierte Sonde bzw. Hybridsonde ausgebildet. Bei dieser Ausführungsform weist die Sonde zusätzlich zu der Elektrode einen Wasserstrahlsondenkörper auf, in dem ein Wasserkanal bis zu einem distalen Ende des Wasserstrahlsondenkörpers verläuft. Der Wasserstrahlsondenkörper erstreckt sich beispielsweise im Wesentlichen parallel zum Sondenkörper mit dem Elektrodenkanal. Der Sondenkörper, der den Elektrodenkanal aufweist, und als Elektrodensondenkörper bezeichnet werden kann, kann gemeinsam mit dem Wasserstrahlsondenkörper in einem Außenkörper, beispielsweise einem Außenrohr oder einem Außenschlauch angeordnet sein. Analog zum Sondenkörper können der Wasserstrahlsondenkörper und/oder der Außenkörper jeweils in Form eines festen Rohrs oder eines flexiblen Schlauchs ausgebildet sein. Der den Elektrodenkanal aufweisende Sondenkörper kann bei einer Ausführungsform auch den Außenkörper bilden, in dem ein Kanal für den Wasserstrahlsondenkörper vorhanden ist.

Bei einer solchen kombinierten Sonde oder Hybridsonde ist der Wasserstrahlsondenkörper entlang seiner Erstreckungsrichtung relativ zum Gehäuse der Bedienvorrichtung und/oder einem Außenkörper verschiebbar angeordnet sein. Die Bedienvorrichtung ist dazu eingerichtet, eine Bewegung des Wasserstrahlsondenkörpers in Richtung einer ausgefahrenen Stellung nur dann zu ermöglichen, wenn sich die Elektrode außerhalb der vollständig ausgefahrenen Position befindet oder wenn sich die Elektrode in der vollständig eingefahrenen Position befindet. Dies kann dadurch erreicht werden, dass der Wasserstrahlsondenkörper mittels der Bedienvorrichtung nur gleichzeitig mit dem Einfahren der Elektrode ausgefahren werden kann oder alternativ erst dann ausgefahren werden kann, wenn die Elektrode teilweise oder vollständig eingefahren ist. Dieser Aspekt der Ausgestaltung der kombinierten Sonde bzw. Hybridsonde kann auch unabhängig davon eingesetzt werden, wie die Elektrodenbedieneinheit mit der Elektrode bewegungsgekoppelt ist, also insbesondere unabhängig davon, ob ein Untersetzungsgetriebe vorhanden ist oder nicht, sowie unabhängig von dem Vorhandensein einer Rasteinrichtung.

Bei einer Ausführungsform weist die Bedienvorrichtung ein Wasserstrahlsonden-Bedienelement auf. Das Wasserstrahlsonden-Bedienelement ist dazu eingerichtet, den Wasserstrahlsondenkörper in der Erstreckungsrichtung relativ zum Gehäuse zu bewegen bzw. zu verschieben. Das Wasserstrahlsonden-Bedienelement ist am Gehäuse der Bedienvorrichtung angeordnet und kann beispielsweise durch einen Schieber gebildet sein.

Das Elektroden-Bedienelement und das Wasserstrahlsonden-Bedienelement können derart benachbart am Gehäuse angeordnet sein, dass das Elektroden-Bedienelement in der ersten Stellung einen Anschlag für das Wasserstrahlsonden-Bedienelement bildet. In der ersten Stellung des Elektroden-Bedienelements ist die Elektrode beispielsgemäß vollständig ausgefahren. In dieser ersten Stellung kann das Wasserstrahlsonden-Bedienelement nicht bewegt werden. Die Bewegung des Wasserstrahlsonden-Bedienelements wird vorzugsweise in die eine Richtung durch das Gehäuse und in die andere Richtung durch das in der ersten Stellung angeordnete Elektroden-Bedienelement blockiert. Das Ausfahren des Wasserstrahlsondenkörpers ist somit verhindert, wenn die Elektrode betrieben wird.

Bei einer anderen bevorzugten Ausführungsform kann das Elektroden-Bedienelement sowohl zur Bewegung der Elektrode, als auch zur Bewegung des Wasserstrahlsondenkörpers eingerichtet sein. Bei dieser Ausführungsform ist das Elektroden-Bedienelement sowohl mit der Elektrode, als auch mit dem Wasserstrahlsondenkörper bewegungsgekoppelt.

Es ist vorteilhaft, wenn das Elektroden-Bedienelement dazu eingerichtet ist, bei einer Bewegung aus der ersten Stellung heraus in Richtung der zweiten Stellung zunächst eine Einfahrbewegung der Elektrode zu bewirken. Erst nach Beginn der Einfahrbewegung oder nach Erreichen der vollständig eingefahrenen Position der Elektrode wird durch fortgesetzte Bewegung des Elektroden-Bedienelements von der ersten Stellung weg eine Ausfahrbewegung des Wasserstrahlsondenkörpers bewirkt. Somit kann das Elektroden-Bedienelement in einem ersten Bewegungsabschnitt unmittelbar im Anschluss an die erste Stellung nur die Elektrode bewegen. In einem sich unmittelbar an die zweite Stellung anschließenden Bewegungsbereich kann das Elektroden-Bedienelement nur eine Bewegung des Wasserstrahlsondenkörpers bewirken. Die beiden Bewegungsbereiche können einen räumlichem Abstand aufweisen, sich direkt aneinander anschließen oder teilweise überlappen.

Es ist außerdem vorteilhaft, wenn im Gehäuses eine Umlenkeinrichtung vorhanden ist, die dazu eingerichtet ist, den Wasserstrahlsondenkörper derart mit einem zugeordneten Bedienelement der Bedienvorrichtung zu koppeln, dass die Bewegungsrichtungen des außerhalb des Gehäuses angeordneten Wasserstrahlsondenkörpers und des Bedienelements gegenläufig zueinander sind. Beispielsweise kann der Wasserstrahlsondenkörper durch eine Umlenkeinrichtung innerhalb des Gehäuses umgelenkt werden. Vorzugsweise lenkt die Umlenkeinrichtung den Wasserstrahlsondenkörper in etwa um 180° um, so dass er im Bereich der Umlenkeinrichtung eine U-förmige Gestalt hat. Durch das Umlenken werden die sich an die Umlenkeinrichtung anschließenden Abschnitte des Wasserstrahlsondenkörpers gegenläufig bewegt. Dadurch wird die Möglichkeit geschaffen, durch eine Bewegung des proximalen Endes des Wasserstrahlsondenkörpers in eine Richtung, eine Bewegung des außerhalb des Gehäuses angeordneten Wasserstrahlsondenkörpers und beispielsweise des distalen Endes des Wasserstrahlsondenkörpers in die entgegengesetzte Richtung zu erreichen.

Vorteilhafte Ausgestaltungen der Erfindung ergeben sich aus den abhängigen Ansprüchen, der Beschreibung und den Zeichnungen. Nachfolgend werden bevorzugte Ausführungsbeispiele der Erfindung anhand der beigefügten Zeichnungen erläutert. Es zeigen:
Figur 1 eine schematische, blockschaltbildähnliche Darstellung eines Ausführungsbeispiels einer Sonde mit ausgefahrener Elektrode und eingefahrenem Wasserstrahlsondenkörper,
Figur 2 eine schematische blockschaltbildähnliche Prinzipdarstellung einer Bedienvorrichtung der Sonde aus Figur 1 mit einem Elektroden-Bedienelement in einer ersten Stellung,
Figur 3 die Bedienvorrichtung aus Figur 2, wobei sich das Elektroden-Bedienelement in einer zweiten Stellung befindet,
Figur 4 die Bedienvorrichtung gemäß der Figuren 2 und 3 in einer abgewandelten Ausführungsform, wobei sich das Elektroden-Bedienelement in einer ersten Stellung befindet,
Figuren 5 bis 7 jeweils eine schematische Prinzipdarstellung einer Umlenkeinrichtung für die Bedienung bzw. Bewegung des Wasserstrahlsondenkörpers der Sonde,
Figur 8 eine schematische Prinzipdarstellung einer Bedienvorrichtung einer Sonde mit einer Rasteinrichtung,
Figur 9 eine perspektivische Teildarstellung eines Ausführungsbeispiels eines Gehäuses einer Bedienvorrichtung einer Sonde mit einer Rasteinrichtung,
Figuren 10-12 eine schematische Prinzipdarstellung eines weiteren Ausführungsbeispiels einer Bedienvorrichtung mit einem Elektroden-Bedienelement, das zur Bewegung der Elektrode sowie des Wasserstrahlsondenkörpers eingerichtet ist, in unterschiedlichen Stellungen,
Figur 13 eine schematische Prinzipdarstellung einer Kopplungseinrichtung der Bedienvorrichtung gemäß der Figuren 10-12,
Figur 14 eine schematische Prinzipdarstellung eines Kopplungskörpers, der mit dem proximalen Ende der Elektrode verbunden ist,
Figur 15 einen distalen Endabschnitt der Elektrode sowie des Sondenkörpers in einer vollständig ausgefahrenen Stellung der Elektrode mit einer im Sondenkörper angeordneten Vorspanneinrichtung,
Figur 16 die Anordnung aus Figur 15 bei vollständig eingefahrener Elektrode,
Figur 17 ein abgewandeltes Ausführungsbeispiel des Sondenkörpers, wobei die Vorspanneinrichtung weiter vom distalen Ende des Sondenkörpers entfernt angeordnet ist,
Figur 18 eine schematische Prinzipdarstellung eines Anschlags für das distale Ende der Elektrode in einem Endstück des Sondenkörpers beispielsgemäß aufweisend eine Vertiefung, in die das distale Ende der Elektrode zumindest teilweise eingreifen kann,
Figur 19 eine Prinzipdarstellung eines Ausführungsbeispiels einer Bedienvorrichtung in Seitenansicht, wobei die Wasserstrahlsonde beispielsweise über ein Druckelement bewegt werden kann,
Figur 20 ein weiteres Ausführungsbeispiel einer Bedienvorrichtung in einer teilgeschnittenen, perspektivischen Ansicht und
Figur 21 ein Ausführungsbeispiel eines Drehmechanismus einer Bedienvorrichtung.

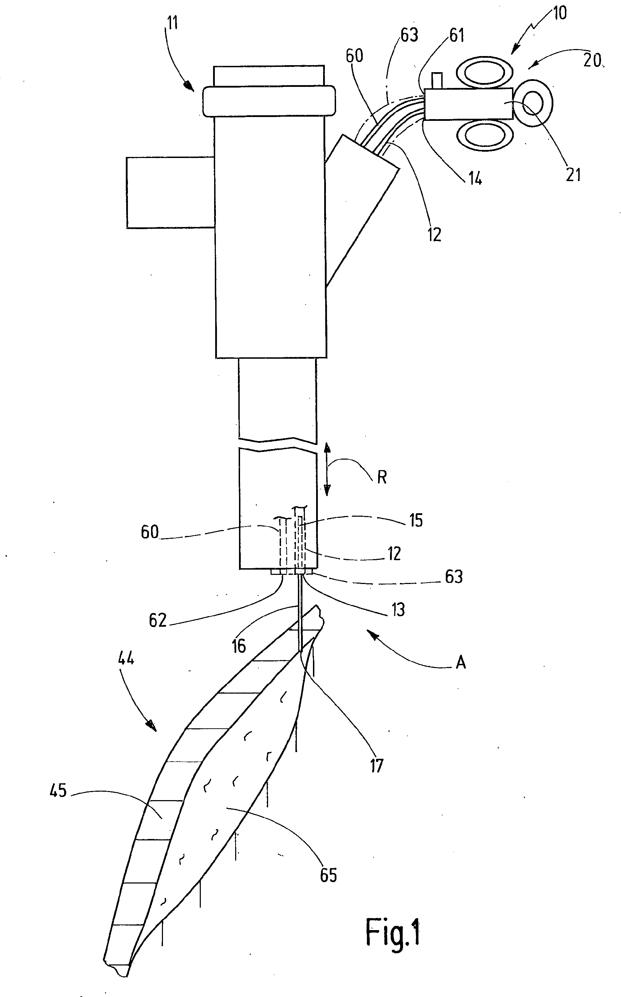

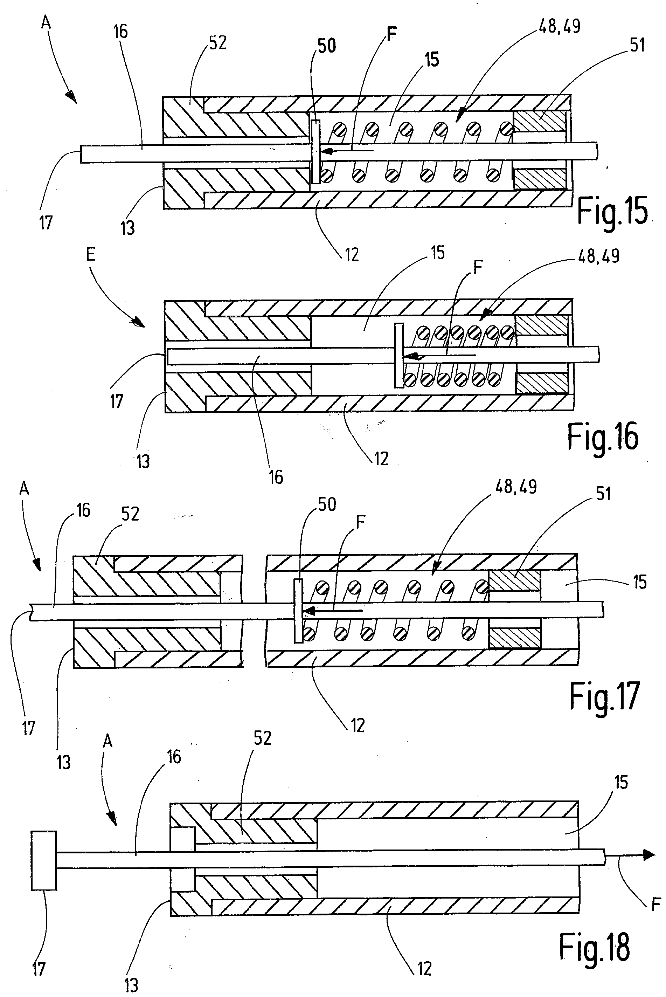

In Figur 1 ist schematisch eine Sonde 10 veranschaulicht, die in Kombination mit einem Endoskop 11 verwendet wird. Die Sonde 10 ist als kombinierte Sonde oder Hybridsonde ausgebildet und weist einen Sondenkörper 12 auf, der ein distales Ende 13 sowie ein proximales Ende 14 hat. Innerhalb des Sondenkörpers 12 erstreckt sich ein Elektrodenkanal 15 (Figuren 15-17), in dem eine Elektrode 16 in einer Erstreckungsrichtung R des Elektrodenkanals 15 verschiebbar angeordnet ist. Die Elektrode 16 kann bis zu einer vollständig ausgefahrenen Position A (Figuren 1, 15, 17, 18) aus dem Sondenkörper 12 herausbewegt werden, wobei in der vollständig ausgefahrenen Position A ein distales Ende 17 der Elektrode 16 den größten Abstand zum distalen Ende 13 des Sondenkörpers 12 hat. In einer vollständig eingefahrenen Position E (Figur 16) befindet sich die Elektrode 16 vorzugsweise innerhalb des Sondenkörpers 12 und das distale Ende 17 der Elektrode 16 ist im Bereich des distalen Endes 13 des Sondenkörpers 12 angeordnet. Bei einer abgewandelten Ausführungsform könnte das distale Ende 17 bei vollständig eingefahrener Position E der Elektrode 16 auch geringfügig aus dem Elektrodenkanal 15 bzw. dem Sondenkörper 12 herausragen.

Der Sondenkörper 12 kann als starres Rohr oder, wie beim Ausführungsbeispiel, als flexibler Schlauch ausgebildet sein. Bei der Ausführung in Form eines flexiblen Schlauchs ist der Sondenkörper 12 bei den im Gebrauch üblicherweise auftretenden Kräften flexibel biegbar und kann insbesondere in Kombination mit einem Endoskop 11 verwendet werden, wie es in Figur 1 dargestellt ist.

Am proximalen Ende 14 ist der Sondenkörper 12 mit einer Bedienvorrichtung 20 verbunden. Die Bedienvorrichtung 20 hat ein Gehäuse 21. Beispielsweise kann der Sondenkörper 12 am Gehäuse 21 befestigt sein und zu einem Innenbereich des Gehäuses 21 offen sein. Die Elektrode 16 kann im Innenbereich des Gehäuses 21 mit einem proximalen Endabschnitt 22 aus dem Elektrodenkanal 15 bzw. dem Sondenkörper 12 herausgeführt sein.

An dem Gehäuse 21 ist ein Elektroden-Bedienelement bewegbar und beispielsgemäß verschiebbar angeordnet. Das Elektroden-Bedienelement 23 hat beim Ausführungsbeispiel zwei Eingreiföffnungen 24, um es mit zwei Fingern ergreifen und beim Ausführungsbeispiel entlang des Gehäuses 21 verschieben zu können.

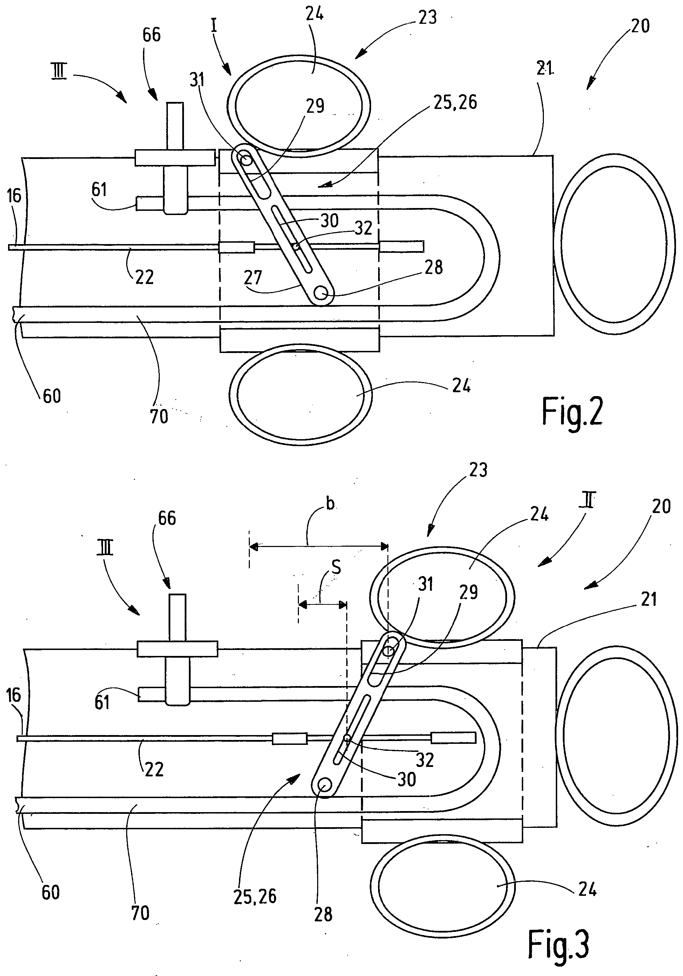

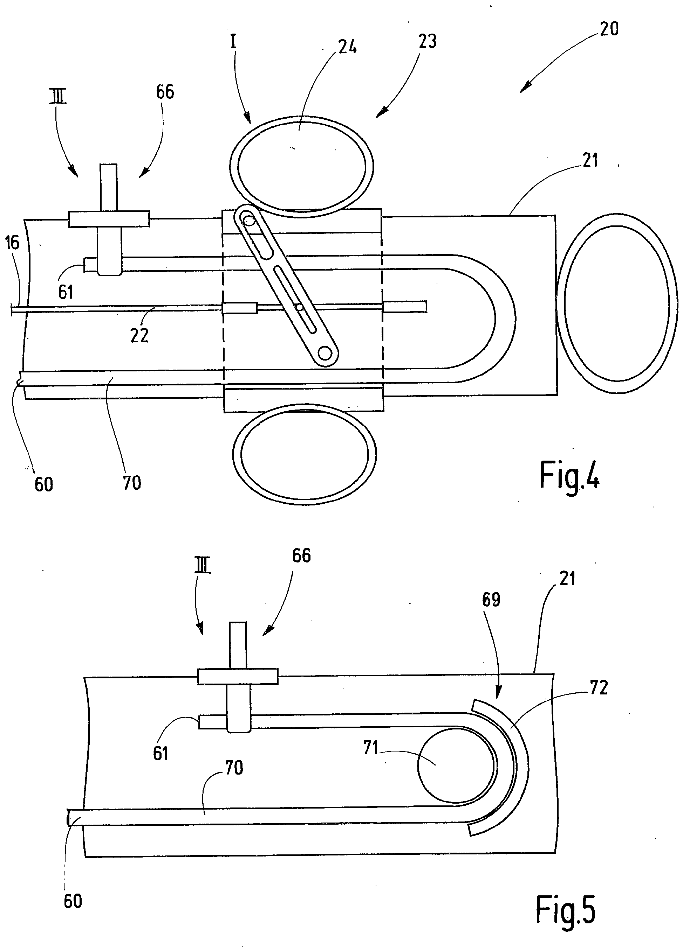

Zur Bewegung der Elektrode 16 zwischen der vollständig ausgefahrenen Stellung A und der vollständig eingefahrenen Stellung E kann das Elektroden-Bedienelement 23 zwischen einer ersten Stellung I (Figuren 2 und 4) und einer zweiten Stellung II bewegt bzw. verschoben werden. Das Elektroden-Bedienelement 23 ist mit der Elektrode 16 bewegungsgekoppelt. In der ersten Stellung I befindet sich die Elektrode 16 in der vollständig ausgefahrenen Position A und in der zweiten Stellung II befindet sich die Elektrode 16 in der vollständig eingefahrenen Position E.

Bei einer bevorzugten Ausführungsform der Sonde 10 bzw. der Bedienvorrichtung 20 ist das Elektroden-Bedienelement 23 mittels eines Untersetzungsgetriebes 25 mit der Elektrode 16 bewegungsgekoppelt. Das Untersetzungsgetriebe 25 ist derart ausgestaltet, dass ein zurückgelegter Bedienweg b des Elektroden-Bedienelements 23 größer ist als ein Weg s, den die Elektrode 16 in Erstreckungsrichtung R zurücklegt. In Figur 3 ist schematisch der maximal zur Verfügung stehende Bedienweg b zwischen der ersten Stellung I und der zweiten Stellung II und der dabei durch die Elektrode 16 zurückgelegte Weg s veranschaulicht.

Es sei an dieser Stelle darauf hingewiesen, dass die Zeichnungen nur Prinzipdarstellungen sind und keine maßstabsgetreuen Abbildungen darstellen. Insbesondere kann das Untersetzungsverhältnis über das Untersetzungsgetriebe 25 ein anderes Untersetzungsverhältnis aufweisen, als es schematisch in Figur 3 durch den Bedienweg b und den Weg s symbolisiert ist.

Beim bevorzugten Ausführungsbeispiel ist das Untersetzungsgetriebe 25 als Hebelgetriebe 26 ausgebildet, insbesondere als einarmiges Hebelgetriebe 26. Das Hebelgetriebe 26 hat einen und vorzugsweise genau einen Hebel 27, der an einer Schwenklagerstelle 28 schwenkbar am Gehäuse 21 angeordnet ist. Die Schwenkachse ist hierbei rechtwinklig zur Bewegungsrichtung des Elektroden-Bedienelements 23 ausgerichtet.

In dem Hebel 27 sind mit Abstand zur Schwenklagerstelle 28 eine erste Nut 29 sowie eine zweite Nut 30 vorhanden. In die erste Nut 29 ragt ein erster Nutenstein 31 hinein, der unbeweglich gegenüber dem Elektroden-Bedienelement 23 am Elektroden-Bedienelement 23 angeordnet ist. In die zweite Nut 30 ragt ein zweiter Nutenstein 32 hinein, der relativ zum proximalen Endabschnitt 22 der Elektrode 16 unbeweglich am proximalen Endabschnitt 22 angeordnet ist. Die beiden Nuten 29, 30 erstrecken sich parallel zueinander und beispielsgemäß jeweils geradlinig und vorzugsweise entlang einer gemeinsamen Geraden, die durch die Schwenklagerstelle 28 verlaufen kann. Die zweite Nut 30 befindet sich beispielsgemäß zwischen der ersten Nut 29 und der Schwenklagerstelle 28. Der zweite Nutenstein 32 ist entlang der zweiten Nut 30 verschiebbar und der erste Nutenstein 31 ist entlang der ersten Nut 29 verschiebbar angeordnet.

Alternativ zu dieser Ausführung mit zwei separaten Nuten 29, 30 könnte auch eine gemeinsame Nut für die Nutensteine 31, 32 vorhanden sein. Somit ist wenigstens eine Nut vorhanden.

Bei einer Bewegung des Elektroden-Bedienelements entlang eines Bedienweges b zwischen der ersten Stellung I und der zweiten Stellung II wird der Hebel 27 um die Schwenklagerstelle 28 geschwenkt. Die Position des ersten Nutensteins 31 in der ersten Nut 29 entspricht einer Kopplungsstelle zwischen dem Elektroden-Bedienelement 23 und dem Untersetzungsgetriebe 25 bzw. dem Hebelgetriebe 26. Die Position des zweiten Nutensteins 32 in der zweiten Nut 30 entspricht einer Kopplungsstelle zwischen der Elektrode 16 bzw. dem proximalen Endabschnitt 22 und dem Untersetzungsgetriebe 25 bzw. Hebelgetriebe 26. Da diese letztere Kopplungsstelle näher an der Schwenklagerstelle 28 angeordnet ist, ist eine Untersetzung durch das Hebelgetriebe 26 bewirkt, wobei gilt: der Bedienweg b ist größer als der Weg s.

Vorzugsweise befindet sich die Schwenklagerstelle 28 an einem Ende des Hebels 27, wie es in den Zeichnungen veranschaulicht ist. In Abwandlung hierzu könnte die Schwenklagerstelle 28 auch zwischen der ersten Nut 29 und der zweiten Nut 30 angeordnet sein, wobei der Abstand zu den Nutensteinen 31, 32 so gewählt wird, dass eine Untersetzung der Bedienbewegung in die Verschiebebewegung der Elektrode 16 erreicht wird.

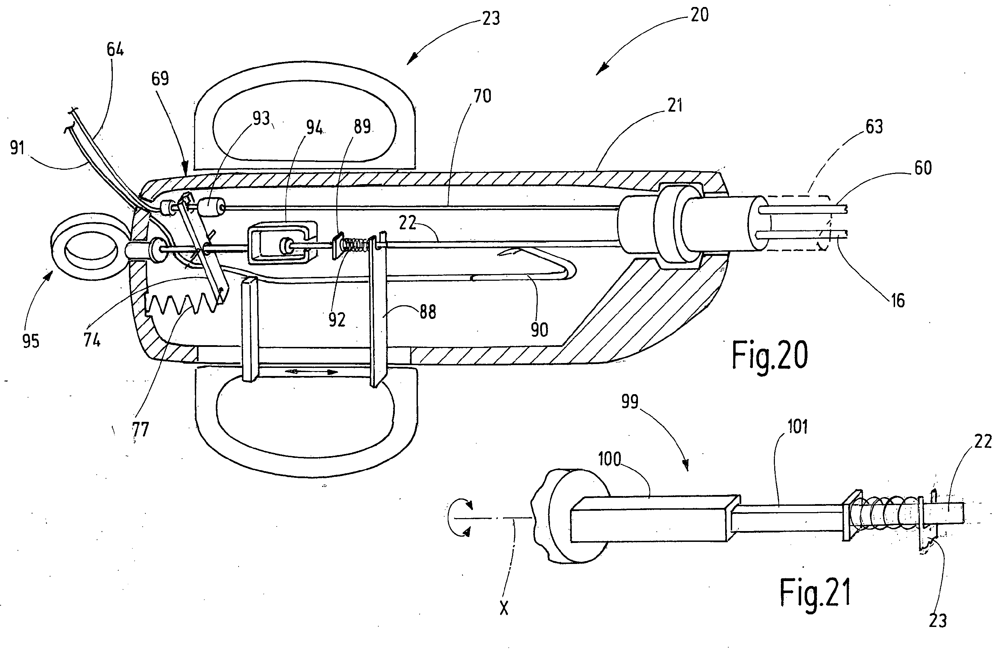

Die Nutensteine 31, 32 können durch Stifte der anderen Vorsprünge gebildet sein. Der Hebel 27 kann auch auf andere Weise mit der Elektrode 16 und dem Elektroden-Bedienelement 23 gekoppelt sein, die eine Bewegung der Kopplungsstellen entlang des Hebels 27 zulässt, wenn dieser geschwenkt wird, beispielsweise eine Kopplung mit einem Hebel, wie sie schematisch in Figur 20 dargestellt ist.

Anstelle des bevorzugten Hebelgetriebes 26 können auch andere Untersetzungsgetriebe 25 verwendet werden, beispielsweise Zahnstangen-Zahnrad-Getriebe, Zahnradgetriebe, Stirnradgetriebe, Reibradgetriebe, Riemengetriebe und dergleichen. Das hier veranschaulichte Hebelgetriebe 26 ist aufgrund der einfachen Ausgestaltung bevorzugt.

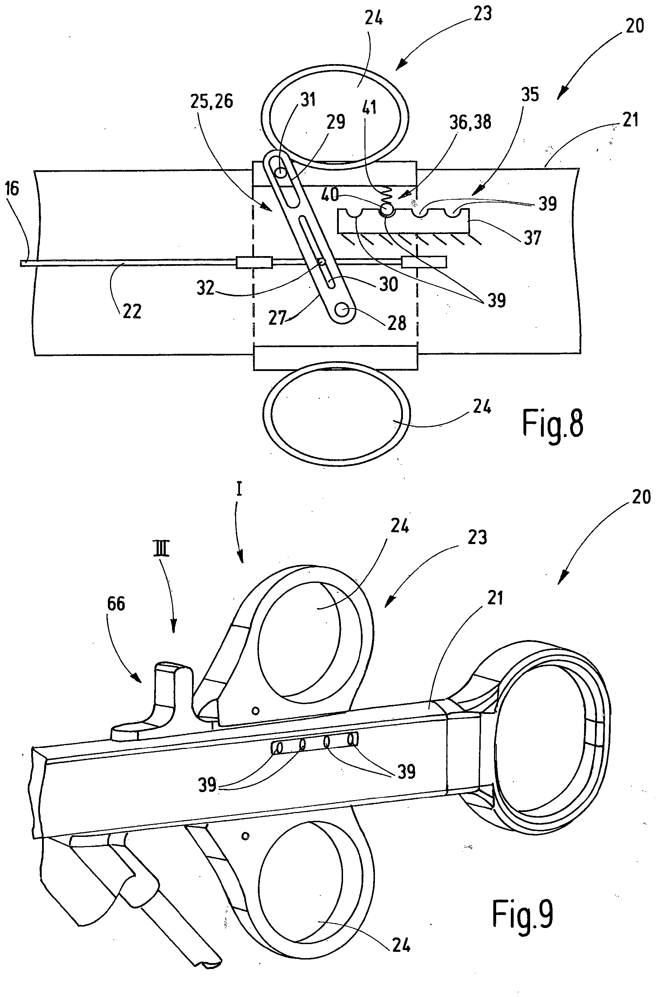

In den Figuren 8 und 9 ist schematisch eine Ausgestaltung der Bedienvorrichtung 20 mit einer Rasteinrichtung 35 veranschaulicht. Die Rasteinrichtung 35 ist dazu eingerichtet, definierte Positionen der Elektrode 16 relativ zum Sondenkörper 12 durch mehrere unterschiedliche Raststellungen vorzugeben. Wegen der Bewegungskopplung des Elektroden-Bedienelements 23 mit der Elektrode 16 über das Untersetzungsgetriebe 25 kann die Rasteinrichtung 35 entweder unmittelbar zwischen dem proximalen Endabschnitt 22 der Elektrode 16 und dem Gehäuse 21 oder, wie beim Ausführungsbeispiel, zwischen dem Elektroden-Bedienelement 23 und dem Gehäuse 21 wirken. Alternativ könnte die Rasteinrichtung 35 auch mit irgendeinem Bestandteil des Untersetzungsgetriebes 25 einerseits und dem Gehäuse 21 andererseits zusammenarbeiten.

Beim Ausführungsbeispiel weist die Rasteinrichtung 35 ein Rastelement 36 auf, das relativ zum Elektroden-Bedienelement 23 unbeweglich am Elektroden-Bedienelement 23 angeordnet ist. Das Rastelement 36 arbeitet mit einem Rastgegenelement 37 zusammen, um unterschiedliche Raststellungen entsprechend der definierten Positionen der Elektrode 16 vorzugeben. Das Rastgegenelement ist beispielsgemäß relativ zum Gehäuse 21 unbeweglich am Gehäuse 21 angeordnet.

Beim Ausführungsbeispiel weist das Rastelement 36 wenigstens einen Rastvorsprung 38 und das Rastgegenelement 37 wenigstens eine Rastausnehmung 39 auf. Umgekehrt könnte das Rastelement 36 auch wenigstens eine Rastausnehmung 39 und das Rastgegenelement 37 wenigstens einen Rastvorsprung 38 aufweisen.

Bei dem hier veranschaulichten Ausführungsbeispiel ist das Rastelement 36 durch einen einzigen Rastvorsprung 38 gebildet. Der Rastvorsprung 38 weist einen Rastkörper 40, beispielsgemäß eine Rastkugel, sowie ein elastisches Lager 41 für den Rastkörper 40 auf. Als elastisches Lager 41 kann beispielsweise eine Feder und insbesondere eine Schraubenfeder verwendet werden, die sich einerseits am Elektroden-Bedienelement 23 abstützt und andererseits den Rastkörper 40 bzw. die Rastkugel trägt. Durch das elastische Lager 41 kann der Rastkörper 40 in eine Rastausnehmung 39 unter einer elastischen Vorspannkraft eingreifen und bei einer Relativbewegung zwischen Rastelement 36 und Rastgegenelement 37 aus der Rastausnehmung 39 gegen die elastische Kraft des elastischen Lagers 41 bewegt werden. Dadurch sind die Raststellungen lösbar und eine Relativbewegung des Elektroden-Bedienelements 23 relativ zum Gehäuse 21 wird durch die Rasteinrichtung 35 nicht übermäßig behindert oder blockiert. Dazu kann die Form und Tiefe der Rastausnehmungen 39 entsprechend gewählt werden.

Bei der hier dargestellten Ausführungsform der Rasteinrichtung 35 definiert eine Raststellung die vollständig ausgefahrene Position A der Elektrode 16, während einer weiteren Raststellung, die vollständig eingefahrenen Position E der Elektrode 16 kennzeichnet. Zwischen diesen beiden Raststellungen ist wenigstens eine weitere Raststellung definiert, die jeweils eine Zwischenposition der Elektrode 16 zwischen der vollständig ausgefahrenen Position A und der vollständig eingefahrenen Position E kennzeichnet. Beispielsweise können drei, vier oder mehr Raststellungen durch die Rasteinrichtung 35 und mithin eine entsprechende Anzahl an Positionen der Elektrode 16 definiert werden.

Die Rasteinrichtung 35 gibt einem Operateur bei der Handhabung der Sonde 10 eine haptische Rückmeldung, wie weit das distale Ende 17 der Elektrode 16 aus dem Sondenkörper 12 herausgefahren ist. Diese Information ist zumindest bei einigen operativen Anwendungen für den Operateur von Bedeutung. Zusätzlich oder alternativ können eine oder mehrere Markierungen vorhanden sein, beispielsweise am Gehäuse 21, die dem Operateur die Stellung des Bedienelements 23 anzeigen, und damit angeben, wie weit das distale Ende 17 der Elektrode 16 aus dem Sondenkörper 12 herausragt.

Wie es schematisch in Figur 1 veranschaulicht ist, ist die Sonde 10 unter Verwendung der Elektrode 16 zur elektrochirurgischen Behandlung von Gewebe 44 eingerichtet und soll dabei insbesondere nur soweit in das Gewebe 44 vordringen, dass eine definierte, äußere Gewebeschicht 45 mittels der Elektrode 16 abgetrennt wird. Die Elektrode 16 dient dabei als Schneidinstrument. Das distale Ende 17 der Elektrode 16 ist für den Operateur nicht sichtbar und befindet sich innerhalb des Gewebes 44. Deshalb ist es für den Operateur wichtig und vorteilhaft, wenn er den Abstand des distalen Endes 17 der Elektrode 16 vom distalen Ende 13 des Sondenkörpers 12 kennt. Hierbei sind die definierten Raststellungen hilfreich.

Wie es in Figur 9 schematisch veranschaulicht ist, kann das Rastgegenelement 37 beispielsweise ein streifenförmiges Element mit Löchern definierter Größe sein, die jeweils eine Rastausnehmung 39 bilden.

Bei einer Ausführungsform der Sonde 10 kann eine Vorspanneinrichtung 48 mit wenigstens einem Vorspannelement 49 vorhanden sein, um eine Vorspannkraft F in Erstreckungsrichtung R auf die Elektrode 16 auszuüben (Figuren 15-18). Das wenigstens eine Vorspannelement 49 kann beispielsweise eine Feder, insbesondere eine Schraubenfeder sein. Bei den in den Figuren 15-17 dargestellten Ausführungsbeispielen ist das wenigstens eine Vorspannelement 49 innerhalb des Elektrodenkanals 15 im Sondenkörper 12 angeordnet. Bei diesen Ausführungsbeispielen übt das wenigstens eine Vorspannelement 49 eine Kraft F auf die Elektrode 16 aus, die die Elektrode 16 in die vollständig ausgefahrene Stellung A drängt. Dazu stützt sich das wenigstens eine Vorspannelement 49 und beispielsgemäß die Schraubenfeder einerseits an einem ersten Abstützteil 50 und andererseits an einem zweiten Abstützteil 51 ab. Das erste Abstützteil 50 ist gegenüber der Elektrode 16 unbeweglich an der Elektrode 16 angeordnet und kann beispielsweise durch einen Flansch, Ringkörper oder dergleichen gebildet sein. Das zweite Abstützteil 51 ist relativ zum Sondenkörper 12 unbeweglich am Sondenkörper 12 angeordnet und kann beispielsweise ein hohlzylindrischer Körper sein, der innen einen Durchgangskanal für die Elektrode 16 bereitstellt und im Elektrodenkanal 15 des Sondenkörpers 12 angeordnet ist. Das wenigstens eine Vorspannelement 49 kann sich somit zwischen den beiden Abstützteilen 50, 51 erstrecken und eine Vorspannkraft F zwischen dem Sondenkörper 12 und der Elektrode 16 bewirken.

Wie es in den Figuren 15 und 16 gezeigt ist, kann das wenigstens eine Vorspannelement 49 unmittelbar im Anschluss an ein Endstück 52 des Sondenkörpers 12 angeordnet sein. Das Endstück 52 kann beispielsweise ein keramisches Endstück sein, das den Sondenkörper 12 gegenüber Funken- und Lichtbildung schützt, wenn an der Elektrode 16 eine Spannung angelegt wird. In der vollständig ausgefahrenen Position A der Elektrode 16 kann das erste Abstützteil 50 an einem inneren Ende des Endstücks 52 anliegen. Eine solche Anlage ist jedoch nicht zwingend erforderlich, wie es auch anhand des Beispiels in Figur 17 veranschaulicht ist.

Bei dem in den Figuren 15 und 16 dargestellten Ausführungsbeispiel ist das wenigstens eine Vorspannelement 49 unmittelbar im Anschluss an das Endstück 52 positioniert, vorzugsweise in einem Bereich mit einer Länge von 10 mm bis 20 mm im Anschluss an das Endstück 52 innerhalb des Elektrodenkanals 15. Bei dem in Figur 17 dargestellten Ausführungsbeispiel ist das wenigstens eine Vorspannelement 49 mit Abstand zum Endstück 52 bzw. zum distalen Ende 13 des Sondenkörpers angeordnet, der größer ist als beim Ausführungsbeispiel gemäß der Figuren 15 und 16. Beispielsweise kann das wenigstens eine Vorspannelement 49 einen Abstand von 10-15 cm zum Endstück 52 bzw. zum distalen Ende 13 des Sondenkörpers 12 aufweisen. Prinzipiell kann das wenigstens eine Vorspannelement 49 an einer beliebigen Stelle innerhalb des Sondenkörpers 12 angeordnet werden, wobei sich Bereiche nahe des proximalen Endes 14 bzw. des distalen Endes 13 anbieten, um das wenigstens eine Vorspannelement 49 bzw. das zweite Abstützteil 51 möglichst einfach innerhalb des Sondenkörpers 12 anordnen zu können.

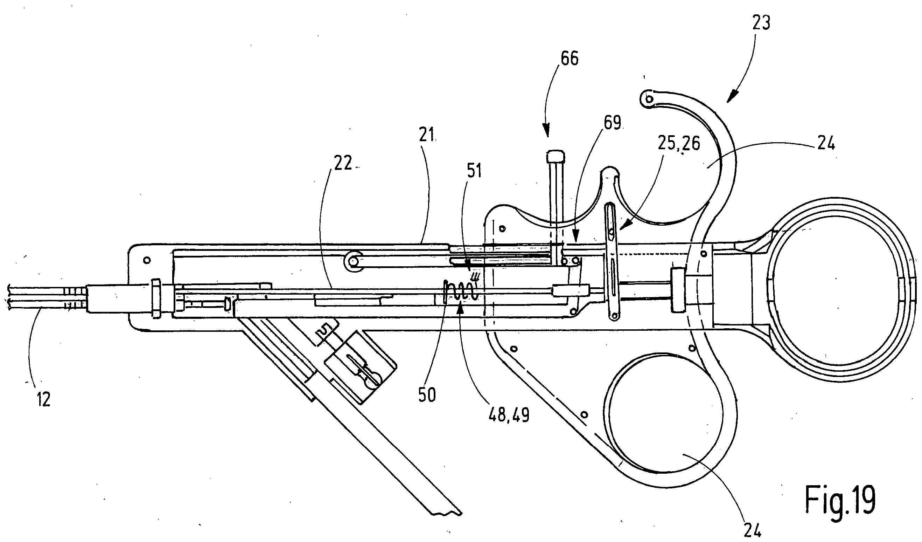

In Abwandlung zu den bisher veranschaulichten Ausführungsbeispielen gemäß der Figuren 15-17, kann die Vorspanneinrichtung 48 bzw. das wenigstens eine Vorspannelement 49 auch außerhalb des Sondenkörpers 12, und insbesondere im Gehäuse 21 angeordnet werden, wie es lediglich stark schematisiert beispielhaft in Figur 19 veranschaulicht ist. Das zweite Abstützteil 51, an dem sich das wenigstens eine Vorspannelement 49 dabei abstützt, ist bei dieser Anordnung relativ zum Gehäuse 21 unbeweglich mit dem Gehäuse 21 verbunden.

In Abwandlung zu den bisher dargestellten Ausführungsbeispielen kann die Vorspanneinrichtung 48 auch eine Vorspannkraft F auf die Elektrode 16 bewirken, die die Elektrode 16 in Richtung ihrer vollständig eingefahrenen Position E drängt, was beispielhaft in Figur 18 dargestellt ist. Bei dieser Ausführungsform kann das wenigstens eine Vorspannelement 49 das distale Ende 17 der Elektrode 16 in Richtung zum distalen Ende 13 des Sondenkörpers 12 drängen (ziehen oder drücken). Auch hierbei können Federn bzw. Schraubenfedern und Anordnungen verwendet werden, analog zur Darstellung der Figuren 15-17 oder 19, wobei z.B. das zweite Abstützteil 51 auf der Seite des ersten Abstützteils 50 angeordnet ist, das sich näher am distalen Ende 17 der Elektrode 16 befindet. Alternativ könnte das wenigstens eine Vorspannelement 49 auch an den Abstützteilen 50, 51 befestigt sein und eine Zugkraft bewirken, indem es die beiden Abstützteile 50, 51 aufeinander zu drängt und nicht wie bei den bisher beschriebenen Ausführungsbeispielen voneinander weg.

Bei der Sonde 10 gemäß der Erfindung handelt es sich um eine kombinierte Sonde bzw. Hybridsonde, die zusätzlich zu der Elektrode 16 für die elektrochirurgische Behandlung einen Wasserstrahlsondenkörper 60 aufweist, der sich von einem proximalen Ende 61 zu einem distalen Ende 62 erstreckt. Der Wasserstrahlsondenkörper 60 und die Elektrode 16 können beim Ausführungsbeispiel auch in einem gemeinsamen Außenkörper 63 angeordnet sein, der beispielsweise durch den Sondenkörper 12 gebildet sein kann und sowohl für den Wasserstrahlsondenkörper 60 als auch für die Elektrode 16 jeweils ein Lumen bzw. einen Kanal bereitstellt.

Der Wasserstrahlsondenkörper 60 weist einen Wasserkanal auf, der sich bis zum distalen Ende 62 erstreckt. Der Wasserkanal ist beispielsweise über eine Zufuhrleitung 64 mit einem Wasserreservoir bzw. einer Druckwasserquelle fluidisch verbindbar. Am distalen Ende 62 des Wasserstrahlsondenkörpers 60 kann ein Wasserstrahl ausgestoßen werden. Mittels dieses Wasserstrahls kann beispielsweise eine äußere Gewebeschicht 45 unterspritzt werden, so dass sich unterhalb der äußeren Gewebeschicht 45 ein Flüssigkeitspolster 65 bildet, das die äußere Gewebeschicht 45 von tieferen Gewebeschichten des Gewebes 44 trennt und das Herausschneiden eines Bereichs, insbesondere eines krankhaft veränderten Bereichs der äußeren Gewebeschicht 45, vereinfacht (Figur 1).

Der Wasserstrahlsondenkörper ist mittels der Bedienvorrichtung 20 in seiner Erstreckungsrichtung R gegenüber dem Gehäuse 21 verschiebbar angeordnet. Analog zur Elektrode 16 kann die Position des distalen Endes 62 des Wasserstrahlsondenkörpers 60 in Erstreckungsrichtung R verändert werden. Beim Ausführungsbeispiel sind die Erstreckungsrichtung R der Elektrode 16 und die Erstreckungsrichtung R des Wasserstrahlsondenkörpers 60 zumindest in dem Abschnitt, der sich an die jeweiligen distalen Enden 17 bzw. 62 anschließt, im Wesentlichen parallel.

Bei einem erfindungsgemäßen Aspekt, der unabhängig von anderen Aspekten realisiert werden kann, ist die Bedienvorrichtung 20 dazu eingerichtet, die Bewegung des Wasserstrahlsondenkörpers 60 in Erstreckungsrichtung R zur ausgefahrenen Position bzw. Stellung nur dann zu ermöglichen, wenn die Elektrode 16 nicht in der vollständig ausgefahrenen Position A ist oder vorzugsweise, wenn sich die Elektrode 16 in der vollständig eingefahrenen Position E befindet.

Bei dem in den Figuren 2 und 3 veranschaulichten Ausführungsbeispiel weist die Bedienvorrichtung 20 zum Verschieben des Wasserstrahlsondenkörpers 60 ein Wasserstrahlsonden-Bedienelement 66 auf. Das Wasserstrahlsonden-Bedienelement 66 ist benachbart zum Elektroden-Bedienelement 23 angeordnet. Das Wasserstrahlsonden-Bedienelement 66 befindet sich in einer Ausgangsstellung III, wenn der Wasserstrahlsondenkörper 60 seine eingefahrene Position (Figur 1) einnimmt. Die Ausgangsstellung III des Wasserstrahlsonden-Bedienelements 66 ist in den Figuren 2 und 3 veranschaulicht.

Beim Ausführungsbeispiel ist das Wasserstrahlsonden-Bedienelement 66 linear verschiebbar am Gehäuse 21 gelagert in eine Richtung parallel zur Verschiebungsrichtung des Elektroden-Bedienelements 23. Befindet sich das Elektroden-Bedienelement 23 in seiner ersten Stellung I, bildet es einen Anschlag für das Wasserstrahlsonden-Bedienelement 66 in dessen Ausgangsstellung III. Ist die Elektrode 16 somit in ihrer vollständig ausgefahrenen Position A, kann das Wasserstrahlsonden-Bedienelement 66 nicht aus der Ausgangsstellung III herausbewegt werden. Diese Situation ist in Figur 2 gezeigt.

Erst wenn das Elektroden-Bedienelement 23 aus der ersten Stellung I wegbewegt wurde, beispielsweise in die zweite Stellung II, wird dadurch dem Wasserstrahlsonden-Bedienelement 66 Bewegungsspielraum gegeben, um es aus der Ausgangsstellung III herauszubewegen und damit dem Wasserstrahlsondenkörper 60 aus der eingefahrenen Position in eine ausgefahrene Position zu bringen (Figur 3). Somit kann bei dem in den Figuren 2 und 3 veranschaulichten Ausführungsbeispiel der Bedienvorrichtung 20 der Wasserstrahlsondenkörper 60 gleichzeitig mit dem Einfahren der Elektrode 16 oder nach dem Einfahren der Elektrode 16 ausgefahren werden.

Die Begriffe "einfahren" und "ausfahren" beziehen sich in der Anmeldung auf den Sondenkörper 12 (bezüglich der Elektrode 16) und den gemeinsamen Außenkörper 63 bzw. das Gehäuse 21 betreffend den Wasserstrahlsondenkörper 60.

Bei einer nicht erfindungsgemäßen alternativen Ausführungsform, die in Figur 4 dargestellt ist, befindet sich das Wasserstrahlsonden-Bedienelement 66 in der Ausgangsstellung III in einem ausreichenden Abstand zu dem Elektroden-Bedienelement 23 in der ersten Stellung I, so dass der Wasserstrahlsondenkörper 60 bei dieser Ausführungsform unabhängig von der Stellung des Elektroden-Bedienelements 23 bewegt werden kann, um den Wasserstrahlsondenkörper 60 ein- und auszufahren.

Anhand der Darstellungen in den Figuren 5-7 sind unterschiedliche Möglichkeiten zur Realisierung einer Umlenkeinrichtung 69 veranschaulicht. Die im Gehäuses 21 angeordnete Umlenkeinrichtung 69 ist dazu eingerichtet, den Wasserstrahlsondenkörper 60 derart mit einem zugeordneten Bedienelement der Bedienvorrichtung 20 zu koppeln, dass die Bewegungsrichtungen des außerhalb des Gehäuses 21 angeordneten Wasserstrahlsondenkörpers 60 und des Bedienelements entgegengesetzt sind.

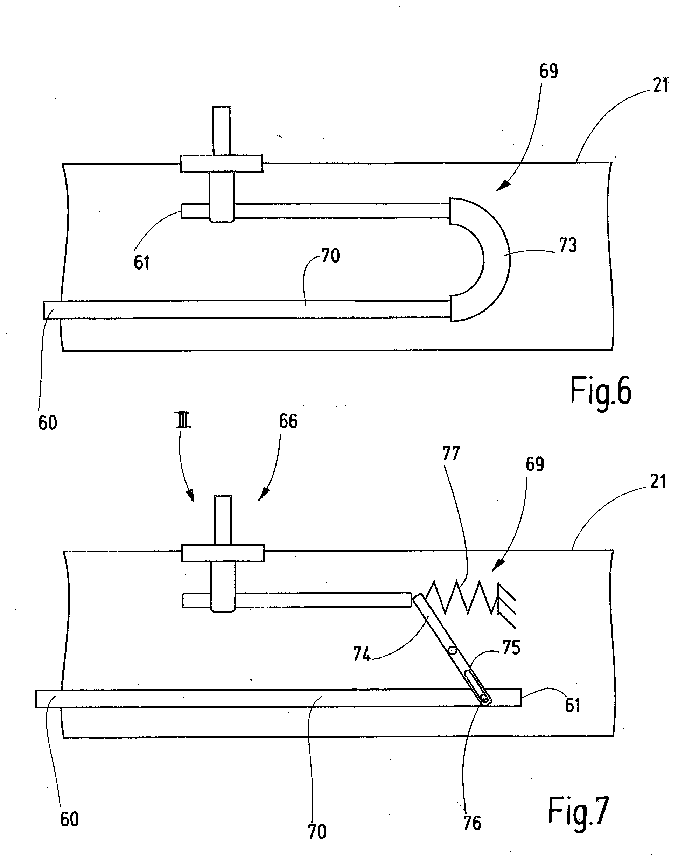

Bei den Ausführungsbeispielen gemäß der Figuren 5 und 6 ist die Umlenkeinrichtung 69 dazu eingerichtet, einen proximalen Endabschnitt 70 des Wasserstrahlsondenkörpers 60, der innerhalb des Gehäuses 21 angeordnet ist, um etwa 180° umzulenken bzw. umzubiegen. Hierzu kann die Umlenkeinrichtung 69 beispielsweise einen mit einem Innenradius gekrümmten inneren Umlenkkörper 71 und einen mit einem äußeren Radius gekrümmten äußeren Umlenkkörper 72 aufweisen, zwischen denen der proximale Endabschnitt 70 des Wasserstrahlsondenkörpers 60 gekrümmt geführt verläuft (Figur 5). Alternativ dazu kann die Umlenkeinrichtung 69 beispielsweise ein gekrümmt verlaufendes Umlenkrohr 73 aufweisen, durch das sich der proximale Endabschnitt 70 erstreckt (Figur 6).

Am proximalen Ende 61, das sich innerhalb des Gehäuses 21 befindet, ist der Wasserstrahlsondenkörper 60 mit dem Wasserstrahlsonden-Bedienelement 66 bewegungsgekoppelt und beispielsgemäß unbeweglich bzw. fest verbunden. Eine Bewegung des Wasserstrahlsonden-Bedienelements 66 führt somit zu einer Verschiebung des Wasserstrahlsondenkörpers 60. Die sich benachbart zur Umlenkeinrichtung 69 erstreckenden Bereiche des proximalen Endabschnitts 70 bewegen sich dabei gegenläufig. Somit kann beispielsweise durch ein Bewegen des Wasserstrahlsonden-Bedienelements 66 aus der Ausgangsstellung III heraus eine Ausfahrbewegung des Wasserstrahlsondenkörpers 60 veranlasst werden und umgekehrt durch ein Bewegen des Wasserstrahlsonden-Bedienelements 66 zurück in Richtung der Ausgangsstellung III eine Einfahrbewegung des Wasserstrahlsondenkörpers 60.

Eine derart entgegengesetzte Bewegung kann auch durch eine andere Art der Umlenkeinrichtung 69 bewirkt werden, wie sie beispielsweise in Figur 7 veranschaulicht ist. Dort weist die Umlenkeinrichtung 69 einen zweiarmigen Hebel 74, der an der Verbindungsstelle seiner beiden Arme schwenkbar am Gehäuse 21 gelagert ist und sich mit einem Arm am Wasserstrahlsonden-Bedienelement 66 und mit dem anderen Arm am proximalen Endabschnitt 70 des Wasserstrahlsondenkörpers 60 abstützt. Der zweiarmige Hebel 74 bzw. das Wasserstrahlsonden-Bedienelement 66 können über eine Feder oder eine andere elastische oder federelastische Einheit 77 in die Ausgangsstellung III vorgespannt sein. Die Verbindung des zweiarmigen Hebels 74 mit dem Wasserstrahlsondenkörper 60 erfolgt über eine dritte Nut 75 im zweiarmigen Hebel 74, in die ein dritter Nutenstein 76 eingreift, der unbeweglich bzw. fest am Wasserstrahlsondenkörper 60 bzw. dessen proximalem Endabschnitt 70 angeordnet ist. Auch mit Hilfe dieses zweiarmigen Hebels 74 lässt sich eine gegenläufige Bewegung erreichen, so dass ein Bewegen des Wasserstrahlsonden-Bedienelements 66 aus der Ausgangsstellung III heraus zu einer Ausfahrbewegung des Wasserstrahlsondenkörpers 60 und umgekehrt ein Bewegen zurück in die Ausgangsstellung III eine Einfahrbewegung des Wasserstrahlsondenkörpers 60 bewirkt.

Das Übersetzungsverhältnis der Umlenkeinrichtung 69 gemäß Figur 7 zwischen der Bewegung des Wasserstrahlsonden-Bedienelements 66 und der Bewegung des Wasserstrahlsondenkörpers 60 kann gleich 1 sein und alternativ wahlweise auch kleiner als 1 oder größer als 1 gewählt werden. Beispielsweise kann das Übersetzungsverhältnis durch die Länge die beiden Arme des zweiarmigen Hebels 74 gewählt werden.

Bei der Ausgestaltung der Umlenkeinrichtung 69 gemäß Figur 7 kann auch eine Ausführungsform der Bedienvorrichtung 20 realisiert werden, bei der auf ein separates Wasserstrahlsonden-Bedienelement 66 verzichtet werden kann. Dabei kann ein einziges Bedienelement und beispielsgemäß das Elektroden-Bedienelement 23 sowohl zum Bewegen der Elektrode 16, als auch zum Bewegen des Wasserstrahlsondenkörpers 60 eingerichtet sein.

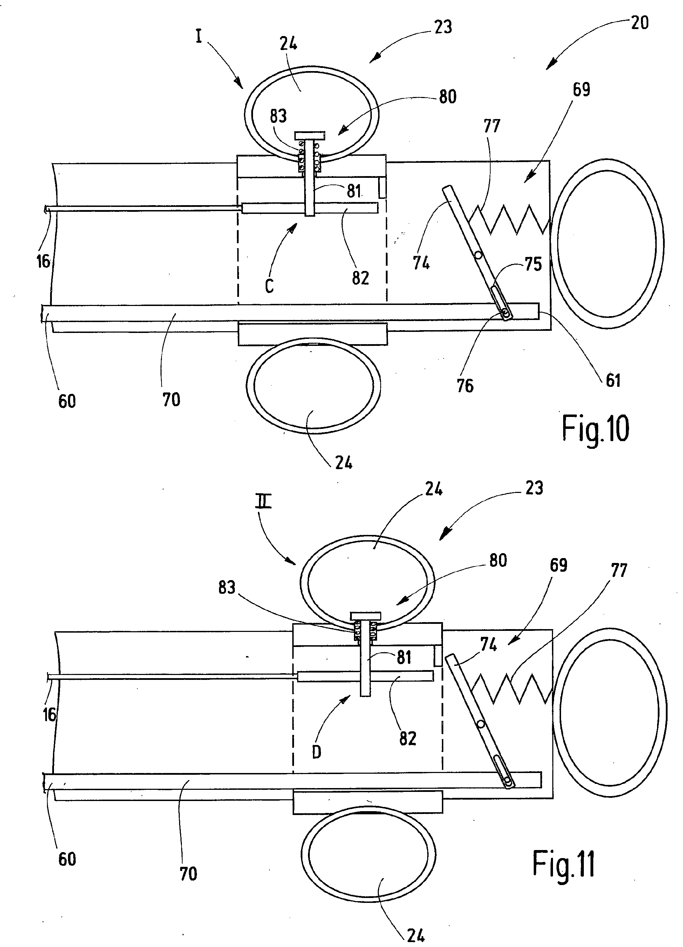

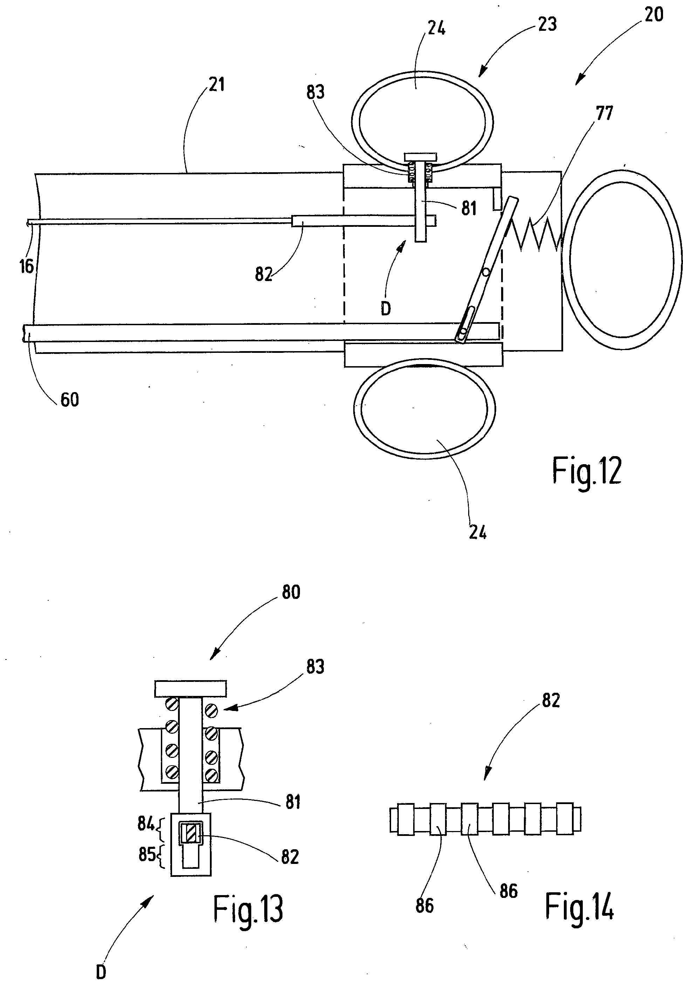

Bei dem in den Figuren 10-12 veranschaulichten Ausführungsbeispiel ist eine Kopplungseinrichtung 80 vorhanden, mittels dem die Bewegungskopplung zwischen dem Elektroden-Bedienelement 23 und der Elektrode 16 hergestellt und getrennt werden kann. Die Kopplungseinrichtung 80 kann zur manuellen Bedienung eingerichtet sein. In einer Kopplungsstellung C besteht eine Bewegungskopplung zwischen der Elektrode 16 und dem Elektroden-Bedienelement 23 (Figur 10) und in einer Entkopplungsstellung D ist die Bewegungskopplung zwischen dem Elektroden-Bedienelement 23 und der Elektrode 16 aufgehoben (Figuren 11 und 12). In der Kopplungsstellung C kann beispielsweise eine kraftschlüssige und/oder formschlüssige Verbindung zwischen einem betätigbaren Kopplungsteil 81 und einem unbeweglich bzw. fest mit der Elektrode 16 verbundenen Kopplungskörper 82 hergestellt sein, während diese kraftschlüssige und/oder formschlüssige Verbindung in der Entkopplungsstellung D, beispielsweise durch eine veränderte Stellung des Kopplungsteils 81, aufgehoben ist. Über ein elastisches oder federelastisches Element 83 kann das Kopplungsteil 81 in die Kopplungsstellung C gedrängt werden. Bei dem federelastischen Element 83 kann es sich beispielsweise um eine Schraubenfeder handeln, die sich einerseits am Kopplungsteil 81 und andererseits am Elektroden-Bedienelement 23 abstützt.

Mittels des Elektroden-Bedienelements 23 kann die Elektrode 16 zunächst aus der ausgefahrenen Position A in Richtung der eingefahrenen Position E bewegt werden, indem das Elektroden-Bedienelement 23 aus der ersten Stellung I heraus in Richtung der zweiten Stellung II bewegt wird (Figur 10). Bei dieser Bewegung wird die Elektrode 16 eingefahren. Vorzugsweise hat die Elektrode 16 ihre vollständig eingefahrene Position E erreicht, bevor das Elektroden-Bedienelement 23 in Kontakt mit der Umlenkeinrichtung 69 und beispielsgemäß dem zweiarmigen Hebel 74 gelangt. Das Elektroden-Bedienelement 23 kann somit die zweite Stellung II einnehmen, bevor es über die Umlenkeinrichtung 69 eine Ausfahrbewegung des Wasserstrahlsondenkörpers 60 bewirkt. Da die Elektrode 16 bereits vollständig eingefahren ist, wird die Kopplungseinrichtung 80 in der zweiten Stellung II des Elektroden-Bedienelements 23 in die Entkopplungsstellung D umgeschaltet, so dass eine weitere Bewegung des Elektroden-Bedienelements 23 möglich ist. Bei dieser fortgesetzten Bewegung von der ersten Stellung I weg über die zweite Stellung II hinaus gelangt das Elektroden-Bedienelement 23 in Kontakt mit der Umlenkeinrichtung 69 und beispielsgemäß dem zweiarmigen Hebel 74 und veranlasst dadurch eine Ausfahrbewegung des Wasserstrahlsondenkörpers 60 (Figur 12).

Die Einfahrbewegung des Wasserstrahlsondenkörpers 60 bzw. die Ausfahrbewegung der Elektrode 16 erfolgen entsprechend in umgekehrter Reihenfolge. Beim Ausführungsbeispiel wird der Wasserstrahlsondenkörper 60 durch die elastische oder federelastische Einheit 77 der Umlenkeinrichtung 69 in die eingefahrene Stellung gedrängt und nimmt diese ein, sobald das Elektroden-Bedienelement 23 nicht mehr in Kontakt mit der Umlenkeinrichtung 69 steht bzw. die zweite Stellung II erreicht hat (Figur 11). Anschließend kann die Bewegungskopplung zwischen der Elektrode 16 und dem Elektroden-Bedienelement 23 über die Kopplungseinrichtung 80 wieder hergestellt werden, so dass ein Ausfahren der Elektrode 16 beim weiteren Bewegen von der zweiten Stellung II in die erste Stellung I möglich ist.

In den Figuren 13 und 14 ist beispielsgemäß eine Ausführung der Kopplungseinrichtung 80 bzw. des Kopplungskörpers 82 veranschaulicht, bei dem eine formschlüssige Verbindung zwischen dem Kopplungsteil 81 und dem Kopplungskörper 82 hergestellt werden kann. Das Kopplungsteil 81 hat hierfür eine Durchgangsöffnung mit einem ersten Bereich 84 und einem zweiten Bereich 85. Die Abmessungen des ersten Bereichs 84 sind so groß, dass keine kraftschlüssige oder formschlüssige Verbindung zwischen dem Kopplungskörper 82 und dem Kopplungsteil 81 erfolgt. Der zweite Bereich 85 ist schmaler als der erste Bereich 84 und kann zwischen zwei Rippen 86 bzw. erweiterte Abschnitte des Kopplungskörpers 82 eingreifen und somit zwischen zwei benachbarten Rippen 86 eine formschlüssige Verbindung zwischen dem Kopplungsteil 81 und dem Kopplungskörper 82 herstellen, wenn sich die Kopplungseinrichtung 80 in der Kopplungsstellung C befindet.

Bei dem in Figur 13 dargestellten Querschnitt befindet sich der Kopplungskörper 82 im ersten Bereich 84 der Durchbrechung, so dass die Kopplungseinrichtung 80 die Entkopplungsstellung D einnimmt. Durch eine Bewegung des Kopplungsteils 81 kann der zweite Bereich 85 zum Umschalten in die Kopplungsstellung C auf Höhe des Kopplungskörpers 82 gebracht werden (in Fig. 13 nach oben), um eine formschlüssige und/oder kraftschlüssige Kopplung zu bewirken.

In Figur 19 ist ein Ausführungsbeispiel der Bedienvorrichtung 20 veranschaulicht, bei dem das Wasserstrahlsonden-Bedienelement 66 über eine Umlenkeinrichtung 69 mit dem Wasserstrahlsondenkörper 60 bewegungsgekoppelt ist. Die Umlenkeinrichtung 69 kann entsprechend dem Ausführungsbeispiel gemäß Figur 7 ausgestaltet sein. Das Elektroden-Bedienelement 23 ist über ein Untersetzungsgetriebe 25 und beispielsgemäß ein Hebelgetriebe gemäß der Figuren 2-4 mit der Elektrode 16 gekoppelt.

In Figur 20 ist eine weitere Ausführungsform der Bedienvorrichtung 20 veranschaulicht, bei der das Elektroden-Bedienelement 23 sowohl zur Bewegung der Elektrode 16, als auch zur Bewegung des Wasserstrahlsondenkörpers 60 eingerichtet ist. Zum Zuführen einer elektrischen Spannung bzw. eines elektrischen Stromes liegt am proximalen Endabschnitt 22 der Elektrode 16 eine Kontaktfeder 90 an, die über eine elektrische Leitung 91 mit einer Spannungs- und/oder Stromquelle elektrisch verbunden sein kann. Eine solche Kontaktierung kann auch bei allen anderen Ausführungsformen der Sonde 10 verwendet werden.

Das Elektroden-Bedienelement 23 ist bei diesem Ausführungsbeispiel über ein elastisches oder federelastisches Verbindungselement 92 mit dem proximalen Endabschnitt 22 der Elektrode 16 bewegungsgekoppelt. Dazu liegt das Elektroden-Bedienelement 23 beispielsweise mittels eines in das Gehäuse 21 hineinragenden Querteils 88 an dem Verbindungselement 92 an, das sich auf der entgegengesetzten Seite an einem unbeweglich mit dem proximalen Endabschnitt 22 der Elektrode 16 verbundenen Anschlagteil 89 abstützt. Das Verbindungselement 92 kann durch eine Schraubenfeder oder ähnliches gebildet sein.

Im Inneren des Gehäuses 21 ist außerdem die Umlenkeinrichtung 69 vorhanden, um die Bewegung des Elektroden-Bedienelements 23 in eine Bewegung des Wasserstrahlsondenkörpers 60 umzusetzen. Die Umlenkeinrichtung 69 und die Bedienung des Wasserstrahlsondenkörpers 60 mittels des Elektroden-Bedienelements 23 entspricht dem Ausführungsbeispiel gemäß der Figuren 10-12, so dass auf die vorstehende Erläuterung Bezug genommen werden kann. Auch beim Ausführungsbeispiel gemäß Figur 20 wird zunächst die Elektrode 16 eingefahren, bevor eine Ausfahrbewegung des Wasserstrahlsondenkörpers 60 stattfindet bzw. umgekehrt.

Anstelle der dritten Nut 75 und des dritten Nutensteins 76 weist der Hebel 74 beim Ausführungsbeispiel gemäß Figur 20 einen Schlitz auf, durch den der proximale Endabschnitt 70 des Wasserstrahlsondenkörpers 60 hindurchverläuft. Benachbart zu dem Schlitz ist ein Anschlagkörper 93 relativ zum proximalen Endabschnitt 70 unbeweglich mit dem proximalen Endabschnitt 70 verbunden, wobei die Abmessungen des Anschlagkörpers 93 größer sind als die des Schlitzes im zweiarmigen Hebel 74. Auch bei dieser Ausgestaltung kann der zweiarmige Hebel 74 über den Anschlagkörper 93 eine Bewegung des Wasserstrahlsondenkörpers 60 veranlassen.

Anstelle der Kopplungseinrichtung 80 ist beim Ausführungsbeispiel gemäß Figur 20 das elastisch verformbare Verbindungselement 92 vorhanden. Sobald das Elektroden-Bedienelement 23 die zweite Stellung II erreicht hat, in der die Elektrode 16 vollständig eingefahren ist, wird das Verbindungselement 92 elastisch verformt und ermöglicht dadurch eine weitere Bewegung des Elektroden-Bedienelements 23, um durch Kontakt mit der Umlenkeinrichtung 69 die Bewegung des Wasserstrahlsondenkörpers 60 durchzuführen. Die Elektrode 16 kann beispielsweise an einem Teil des Gehäuses 21 oder des Sondenkörpers 12 anliegen - beispielsweise kann das Anschlagteil 89 am Gegenanschlags 94 und/oder ein erweitertes distales Ende 17 an einem Endstück 52 (Figur 18) anliegen - stoppt die weitere Einfahrbewegung der Elektrode 16 und eine fortgesetzte Bewegung des Elektroden-Bedienelements 23 führt zum elastischen Verformen des Verbindungselements 92. Im Bewegungsbereich zwischen der ersten Stellung I und der zweiten Stellung II des Elektroden-Bedienelements 23 bleibt der Wasserstrahlsondenkörper 60 in der eingefahrenen Stellung und es wird lediglich die Elektrode 16 eingefahren bzw. ausgefahren.

Optional und abhängig von der Ausgestaltung der Elektrode 16 kann ein verstellbarer Gegenanschlag 94 für das Anschlagteil 89 vorhanden sein. Mittels der Position des Gegenanschlags 94 kann die zweite Stellung II definiert werden. Sobald die Elektrode 16 an einem Anschlag anliegt - beispielsweise das Anschlagteil 89 am Gegenanschlags 94 und/oder ein erweitertes distales Ende 17 an einem Endstück 52 (Figur 18) - stoppt eine weitere Einfahrbewegung der Elektrode 16 und eine fortgesetzte Bewegung des Elektroden-Bedienelements 23 führt zum elastischen Verformen des Verbindungselements 92. Zur Verstellung der Position des Gegenanschlags 94 kann beispielsweise eine drehbare Justageschraube 95 vorhanden sein, die über eine Gewindeverbindung mit dem Gegenanschlag 94 gekoppelt ist und bei einer Drehung die Position des Gegenanschlags 94 parallel zur Bewegungsrichtung des Elektroden-Bedienelements 23 variiert.

In Figur 21 ist eine weitere optionale Ausgestaltungsmöglichkeit der Bedienvorrichtung 20 veranschaulicht, die zusätzlich einen Drehmechanismus 99 veranschaulicht, um eine Elektrode 16 um ihre eigene Längsachse bzw. Erstreckungsachse drehen zu können, beispielsweise wenn das distale Ende 17 der Elektrode 16 nicht rotationssymmetrisch ausgestaltet ist. Der Drehmechanismus 99 kann ein um eine Drehachse X drehbares vorzugsweise unrundes Drehteil 100, beispielsweise ein Rohr mit unrundem Innenquerschnitt, und ein vorzugsweise unrundes Teleskopteil 101, beispielsweise ein Einschubteil, aufweisen. Das Drehteil 100 und das Teleskopteil 101 sind teleskopartig verschiebbar und drehfest miteinander verbunden ist. Beispielsweise ist das Teleskopteil 101 (Einschubteil) in das unrunde Drehteil 100 einschiebbar, wobei der Querschnitt des Teleskopteils 101 im Wesentlichen dem Innenquerschnitt des rohrförmigen Drehteils 100 entsprechen kann. Bei einer Drehung des rohrförmigen Drehteils 100 um die Drehachse X, dreht sich auch das Teleskopteil 101 um die Drehachse X. Das Teleskopteil 101 ist wiederum drehfest mit der Elektrode 16 bzw. dem proximalen Endabschnitt 22 verbunden.

Das Drehteil 100 und/oder das Teleskopteil 101 können teleskopartig parallel zur Drehachse X bzw. entlang der Drehachse X verschiebbar angeordnet sein, um die Elektrode 16 ein- und ausfahren zu können. Das Drehteil 100 kann mit einem von außen zugänglichen Bedienelement am Gehäuse 21 drehfest oder antriebsverbunden sein.

Die Erfindung betrifft die Sonde 10 als kombinierte Sonde bzw. Hybridsonde ausgebildet ist und einen Wasserstrahlsondenkörper 60 aufweist, der im Wesentlichen parallel zum Sondenkörper 12 verläuft und mittels der Bedienvorrichtung 20 in Erstreckungsrichtung R bewegbar bzw. verschiebbar ist. Dabei kann die Bedienvorrichtung 20 derart ausgestaltet sein, dass eine Ausfahrbewegung des Wasserstrahlsondenkörpers nur dann möglich ist, wenn die Elektrode 16 eingefahren wird oder sich bereits in der vollständig eingefahrenen Position E befindet. Ein vorteilhafter Aspekt betrifft eine Sonde 10 zur elektrochirurgischen Behandlung von Gewebe 44. In einem Sondenkörper 12 ist in einer Erstreckungsrichtung R eines Elektrodenkanals 15 eine Elektrode 16 bewegbar angeordnet. Der Sondenkörper 12 ist am proximalen Ende 14 mit einer Bedienvorrichtung 20 verbunden. Diese weist ein Elektroden-Bedienelement 23 auf, um die Elektrode 16 entlang des Elektrodenkanals 15 zu verschieben. Die Bewegungskopplung zwischen dem Elektroden-Bedienelement 23 und der Elektrode 16 erfolgt über ein Untersetzungsgetriebe 25, beispielsweise ein Hebelgetriebe 26. Bei einem weiteren vorteilhaften Aspekt ist eine Rasteinrichtung 35 vorhanden, die die zwei, drei oder mehr lösbare Raststellungen definiert, die jeweils einer vorgegebenen Position der Elektrode 16 relativ zum Sondenkörper 12 entsprechen.

### Bezugszeichenliste:

- 10: Sonde
- 11: Endoskop
- 12: Sondenkörper
- 13: distales Ende des Sondenkörpers
- 14: proximales Ende des Sondenkörpers
- 15: Elektrodenkanal
- 16: Elektrode
- 17: distales Ende der Elektrode

- 20: Bedienvorrichtung
- 21: Gehäuse
- 22: proximaler Endabschnitt der Elektrode
- 23: Elektroden-Bedienelement
- 24: Eingreiföffnung
- 25: Untersetzungsgetriebe
- 26: Hebelgetriebe
- 27: Hebel
- 28: Schwenklagerstelle
- 29: erste Nut
- 30: zweite Nut
- 31: erster Nutenstein
- 32: zweiter Nutenstein

- 35: Rasteinrichtung
- 36: Rastelement
- 37: Rastgegenelement
- 38: Rastvorsprung
- 39: Rastausnehmung
- 40: Rastkörper
- 41: elastisches Lager
- 44: Gewebe
- 45: äußere Gewebeschicht

- 48: Vorspanneinrichtung
- 49: Vorspannelement
- 50: erstes Abstützteil
- 51: zweites Abstützteil
- 52: Endstück

- 60: Wasserstrahlsondenkörper
- 61: proximales Ende des Wasserstrahlsondenkörpers
- 62: distales Ende des Wasserstrahlsondenkörpers
- 63: Außenkörper
- 64: Zufuhrleitung
- 65: Flüssigkeitspolster
- 66: Wasserstrahlsonden-Bedienelement

- 69: Umlenkeinrichtung
- 70: proximaler Endabschnitt des Wasserstrahlsondenkörpers
- 71: innerer Umlenkkörper
- 72: äußerer Umlenkkörper
- 73: Umlenkrohr
- 74: zweiarmiger Hebel
- 75: dritte Nut
- 76: dritter Nutenstein
- 77: elastische oder federelastische Einheit

- 80: Kopplungseinrichtung
- 81: Kopplungsteil
- 82: Kopplungskörper
- 83: elastisches oder federelastisches Element
- 84: erster Bereich der Durchgangsöffnung
- 85: zweiter Bereich der Durchgangsöffnung
- 86: Rippe

- 88: Querteil
- 89: Anschlagteil
- 90: Kontaktfeder
- 91: elektrische Leitung
- 92: Verbindungselement
- 93: Anschlagkörper
- 94: verstellbarer Gegenanschlag
- 95: Justageschraube

- 99: Drehmechanismus
- 100: Drehteil
- 101: Teleskopteil

- I: erste Stellung
- II: zweite Stellung
- III: Ausgangsstellung

- A: vollständig ausgefahrene Position
- b: Bedienweg
- C: Kopplungsstellung
- D: Entkopplungsstellung
- E: vollständig eingefahrene Position
- F: Vorspannkraft
- R: Erstreckungsrichtung
- s: Weg
- X: Drehachse

## Patentansprüche

1. Sonde (10) zur elektrochirurgischen Behandlung von Gewebe (44),
mit einem Sondenkörper (12), in dem sich ein Elektrodenkanal (15) bis zu einem distalen Ende (13) des Sondenkörpers (12) erstreckt,
mit einer ein Gehäuse (21) aufweisenden Bedienvorrichtung (20), die mit einem proximalen Ende (14) des Sondenkörpers (12) verbunden ist, und die am Gehäuse (21) ein Elektroden-Bedienelement (23) aufweist, das zwischen einer ersten Stellung (I) und einer zweiten Stellung (II) bewegbar ist,
mit einer in dem Elektrodenkanal (15) in einer Erstreckungsrichtung (R) des Elektrodenkanals (15) verschiebbar angeordneten Elektrode (16), mit dem Elektroden-Bedienelement (23) bewegungsgekoppelt ist,
mit einem Wasserstrahlsondenkörper (60), in dem sich ein Wasserkanal bis zu einem distalen Ende (62) des Wasserstrahlsondenkörpers (60) erstreckt, wobei der Wasserstrahlsondenkörper (60) in seiner Erstreckungsrichtung (R) relativ zum Gehäuse (21) der Bedienvorrichtung (20) verschiebbar ist,
**dadurch gekennzeichnet, dass** die Bedienvorrichtung (20) dazu eingerichtet ist, eine Bewegung des Wasserstrahlsondenkörpers (60) in Richtung einer ausgefahrenen Stellung nur zu ermöglichen, wenn sich die Elektrode (16) außerhalb der vollständig ausgefahrenen Position (A) befindet oder wenn sich die Elektrode (16) in einer vollständig eingefahrenen Position (E) befindet.

2. Sonde nach Anspruch 1,
**dadurch gekennzeichnet, dass** die Elektrode (16) mittels eines Untersetzungsgetriebes (25) mit dem Elektroden-Bedienelement (23) bewegungsgekoppelt ist, so dass ein zurückgelegter Bedienweg (b) des Elektroden-Bedienelements (23) größer ist als der Weg (s), den die Elektrode (16) dabei im Elektrodenkanal (15) zurücklegt.

3. Sonde nach Anspruch 2,
**dadurch gekennzeichnet, dass** das Untersetzungsgetriebe (25) ein Hebelgetriebe (26) ist und dass das Hebelgetriebe (26) einen an einer Schwenklagerstelle (28) schwenkbar am Gehäuse (21) der Bedienvorrichtung (20) gelagerten Hebel (27) aufweist, wobei eine Kopplungsstelle zwischen dem Hebel (27) und dem Elektroden-Bedienelement (23) weiter von der Schwenklagerstelle (28) entfernt ist als eine Kopplungsstelle zwischen dem Hebel (27) und der Elektrode (16).

4. Sonde nach einem der vorhergehenden Ansprüche,
**dadurch gekennzeichnet, dass** eine Rasteinrichtung (35) vorhanden ist, die unterschiedliche Positionen der Elektrode (16) relativ zum Sondenkörper (12) durch jeweils eine Raststellung definiert.

5. Sonde nach Anspruch 4,
**dadurch gekennzeichnet, dass** die Rasteinrichtung (35) eine vollständig ausgefahrene Position (A) der Elektrode (16), eine vollständig eingefahrene Position (E) der Elektrode (16) und wenigstens eine Zwischenposition der Elektrode (16) zwischen der vollständig ausgefahrenen Position (A) und der vollständig eingefahrenen Position (E) definiert.

6. Sonde nach Anspruch 4 oder 5,
**dadurch gekennzeichnet, dass** die Rasteinrichtung (35) ein mit dem Elektroden-Bedienelement (23) bewegungsverbundenes Rastelement (36) und ein mit dem Gehäuse (21) bewegungsverbundenes Rastgegenelement (37) aufweist, die in mehreren verschiedenen Relativpositionen eine lösbare Rastverbindung einnehmen.

7. Sonde nach Anspruch 6,
**dadurch gekennzeichnet, dass** das Rastelement (36) wenigstens einen Rastvorsprung (38) aufweist und das Rastgegenelement (37) mehrere Rastausnehmungen (39) aufweist oder dass das Rastelement (36) mehrere Rastvorsprünge (38) aufweist und das Rastgegenelement (37) wenigstens eine Rastausnehmung (39) aufweist.

8. Sonde nach einem der vorhergehenden Ansprüche,
**dadurch gekennzeichnet, dass** eine Vorspanneinrichtung (48) vorhanden ist, die die Elektrode (16) relativ zum Sondenkörper (12) in Erstreckungsrichtung (R) mit einer Vorspannkraft (F) beaufschlagt.

9. Sonde nach Anspruch 8,
**dadurch gekennzeichnet, dass** die Vorspannkraft (F) der Vorspanneinrichtung (48) die Elektrode (16) in Richtung einer vollständig ausgefahrenen Stellung (A) drängt.

10. Sonde nach Anspruch 8 oder 9,
**dadurch gekennzeichnet, dass** das Vorspanneinrichtung (48) im Sondenkörper (12) und/oder im Gehäuse (21) angeordnet ist.

11. Sonde nach einem der vorhergehenden Ansprüche,
**dadurch gekennzeichnet, dass** die Bedienvorrichtung (20) ein Wasserstrahlsonden-Bedienelement (66) aufweist, das mit dem Wasserstrahlsondenkörper (60) bewegungsgekoppelt und dazu eingerichtet ist, den Wasserstrahlsondenkörper (60) in seiner Erstreckungsrichtung (R) relativ zum Gehäuse (21) der Bedienvorrichtung (20) zu verschieben.

12. Sonde nach Anspruch 11,
**dadurch gekennzeichnet, dass** das Elektroden-Bedienelement (23) in der ersten Stellung (I) einen Anschlag für das Wasserstrahlsonden-Bedienelement (66) darstellt.

13. Sonde nach einem der Ansprüche 1 bis 10,
**dadurch gekennzeichnet, dass** das Elektroden-Bedienelement (23) sowohl zur Bewegung der Elektrode (16) als auch zur Bewegung des Wasserstrahlsondenkörpers (60) eingerichtet ist.

14. Sonde nach Anspruch 13,
**dadurch gekennzeichnet, dass** das Elektroden-Bedienelement (23) dazu eingerichtet ist, bei einer Bewegung aus der ersten Stellung (I) in Richtung der zweiten Stellung (II) zunächst eine Einfahrbewegung der Elektrode (16) zu bewirken und anschließend eine Ausfahrbewegung des Wasserstrahlsondenkörpers (60) zu bewirken.

15. Sonde nach einem der vorhergehenden Ansprüche,
**dadurch gekennzeichnet, dass** im Gehäuses (21) eine Umlenkeinrichtung (69) vorhanden ist, die dazu eingerichtet ist, den Wasserstrahlsondenkörper (60) derart mit einem zugeordneten Bedienelement (23, 66) der Bedienvorrichtung (20) zu koppeln, dass die Bewegungsrichtungen des außerhalb des Gehäuses (21) angeordneten Wasserstrahlsondenkörpers (60) und des Bedienelements (23, 66) gegenläufig zueinander sind.

## Claims

1. A probe (10) for electrosurgical treatment of tissue (44), comprising:
a probe body (12) in which an electrode channel (15) extends up to a distal end (13) of the probe body (12),
an operating device (20) comprising a housing (21), wherein the operating device (20) is connected to a proximal end (14) of the probe body (12) and comprises an electrode operating element (23) on the housing (21), which is movable between a first position (I) and a second position (II),
an electrode (16) which is arranged to be shiftable in the electrode channel (15) in an extension direction (R) of the electrode channel (15), movably coupled to the electrode operating element (23),
a water jet probe body (60) in which a water channel extends up to a distal end (62) of the water jet probe body (60), wherein the water jet probe body (60) is shiftable in its extension direction (R) relative to the housing (21) of the operating device (20),
**characterized in that** the operating device (20) is configured to allow a movement of the water jet probe body (60) in the direction of an extended position only when the electrode (16) is outside of the fully extended position (A) or when the electrode (16) is in a fully retracted position (E).

2. The probe according to claim 1,
**characterized in that** the electrode (16) is movably coupled to the electrode operating element (23) by means of a reduction gear (25), so that a travelled operating path (b) of the electrode operating element (23) is larger than the path (s) that the electrode (16) thereby travels in the electrode channel (15).

3. The probe according to claim 2,
**characterized in that** the reduction gear (25) is a lever gear (26) and that the lever gear (26) comprises a lever (27) that is pivotably mounted on the housing (21) of the operating device (20) at a pivot location, wherein a coupling location between the lever (27) and the electrode operating element (23) is further away from the pivot location (28) than a coupling location between the lever (27) and the electrode (16).

4. The probe according to anyone of the preceding claims, **characterized in that** a latch device (35) is provided which defines different positions of the electrode (16) relative to the probe body (12) by means of a respective latch position.

5. The probe according to claim 4,
**characterized in that** the latch device (35) defines a fully extended position (A) of the electrode (16), a fully retracted position (E) of the electrode (16) and at least one intermediate position of the electrode (16) between the fully extended position (A) and the fully retracted position (E).

6. The probe according to claim 4 or 5,
**characterized in that** the latch device (35) comprises a latch element (36) which is movably coupled to the electrode operating element (23) and a latch counter element (37) which is movably coupled to the housing (21), which assume a releasable latched connection in a plurality of different relative positions.

7. The probe according to claim 6,
**characterized in that** the latch element (36) comprises at least one latch projection (38) and the latch counter element (37) comprises a plurality of latch recesses (39) or that the latch element (36) comprises a plurality of latch projections (38) and the latch counter element (37) comprises at least one latch recess (39).

8. The probe according to anyone of the preceding claims, **characterized in that** a biasing device (48) is provided which applies a biasing force (F) to the electrode (16) relative to the probe body (12) in the extension direction (R).

9. The probe according to claim 8,
**characterized in that** the biasing force (F) of the biasing device (48) urges the electrode (16) in a direction toward a fully extended position (A).

10. The probe according to claim 8 or 9,
**characterized in that** the biasing device (48) is arranged in the probe body (12) and/or in the housing (21).

11. The probe according to anyone of the preceding claims, **characterized in that** the operating device (20) comprises a water jet probe operating element (66) which is movably coupled to the water jet probe body (60) and is configured to shift the water jet probe body (60) in its extension direction (R) relative to the housing (21) of the operating device (20).

12. The probe according to claim 11,
**characterized in that** the electrode operating element (23) provides a stop for the water jet probe operating element (66) in the first position (I).

13. The probe according to anyone of claims 1 to 10, **characterized in that** the electrode operating element (23) is configured both for moving the electrode (16) and for moving the water jet probe body (60).

14. The probe according to claim 13,
**characterized in that** the electrode operating element (23) is configured to first cause a retraction movement of the electrode (16) and then to cause an extension movement of the water jet probe body (60) during a movement from the first position (I) in the direction toward the second position (II).

15. The probe according to anyone of the preceding claims, **characterized in that** a deflection device (69) is provided in the housing (21), which is configured to couple the water jet probe body (60) to an associated operating element (23, 66) of the operating device (20) such that the movement directions of the water jet probe body (60) arranged outside the housing (21) and of the operating element (23, 66) are opposite to one another.

## Revendications

1. Sonde (10) destinée au traitement électrochirurgical de tissus (44),
comprenant un corps de sonde (12) dans lequel un conduit d'électrode (15) s'étend jusqu'à une extrémité distale (13) du corps de sonde (12),
comprenant un dispositif de commande (20) qui présente un boîtier (21) et est relié à une extrémité proximale (14) du corps de sonde (12) et qui présente, sur le boîtier (21), un élément de commande d'électrode (23) pouvant être déplacé entre une première position (I) et une deuxième position (II),
comprenant une électrode (16) qui est disposée dans le conduit d'électrode (15) avec possibilité de coulissement dans le sens de l'étendue (R) du conduit d'électrode (15), en étant couplée en mouvement à l'élément de commande d'électrode (23),
comprenant un corps de sonde à jet d'eau (60) dans lequel un conduit d'eau s'étend jusqu'à une extrémité distale (62) du corps de sonde à jet d'eau (60), le corps de sonde à jet d'eau (60) pouvant être déplacé dans le sens de son étendue (R) par rapport au boîtier (21) du dispositif de commande (20),
**caractérisée en ce que** le dispositif de commande (20) est conçu pour permettre un mouvement du corps de sonde à jet d'eau (60) dans le sens d'une position déployée, uniquement si l'électrode (16) se trouve à l'extérieur de la position (A) complètement déployée ou si l'électrode (16) se trouve dans une position (E) complètement rentrée.

2. Sonde selon la revendication 1, **caractérisée en ce que** l'électrode (16) est couplée en mouvement à l'élément de commande d'électrode (23) à l'aide d'un mécanisme réducteur (25), de sorte qu'une course de commande (b) parcourue par l'élément de commande d'électrode (23) est plus grande que la course (s) parcourue à cet effet par l'électrode (16) dans le conduit d'électrode (15).

3. Sonde selon la revendication 2, **caractérisée en ce que** le mécanisme réducteur (25) est un mécanisme à levier (26) et **en ce que** le mécanisme à levier (26) présente un levier (27) qui est monté pivotant sur le boîtier (21) du dispositif de commande (20), en un point de palier pivotant (28), sachant qu'un point de couplage entre le levier (27) et l'élément de commande d'électrode (23) est plus éloigné du point de palier pivotant (28) qu'un point de couplage entre le levier (27) et l'électrode (16).

4. Sonde selon une des revendications précédentes, **caractérisée en ce qu'**il est prévu un dispositif d'encliquetage (35) qui définit des positions différentes de l'électrode (16) par rapport au corps de sonde (12), respectivement par le biais d'une position d'encliquetage.

5. Sonde selon la revendication 4, **caractérisée en ce que** le dispositif d'encliquetage (35) définit une position (A) complètement déployée de l'électrode (16), une position (E) complètement rentrée de l'électrode (16) et au moins une position intermédiaire de l'électrode (16), entre la position (A) complètement déployée de l'électrode (16) et la position (E) complètement rentrée.

6. Sonde selon la revendication 4 ou 5, **caractérisée en ce que** le dispositif d'encliquetage (35) présente un élément d'encliquetage (36), lié en mouvement à l'élément de commande d'électrode (23), et un élément d'encliquetage conjugué (37) lié en mouvement au boîtier (21), qui occupent une liaison d'encliquetage libérable dans plusieurs positions relatives différentes.

7. Sonde selon la revendication 6, **caractérisée en ce que** l'élément d'encliquetage (36) présente au moins une saillie d'encliquetage (38) et l'élément d'encliquetage conjugué (37) présente plusieurs évidements d'encliquetage (39), ou **en ce que** l'élément d'encliquetage (36) présente plusieurs saillies d'encliquetage (38) et l'élément d'encliquetage conjugué (37) présente au moins un évidement d'encliquetage (39).

8. Sonde selon une des revendications précédentes, **caractérisée en ce qu'**il existe un dispositif de précontrainte (48) qui applique une force de précontrainte (F) à l'électrode (16) par rapport au corps de sonde (12), dans le sens de l'étendue (R).

9. Sonde selon la revendication 8, **caractérisée en ce que** la force de précontrainte (F) du dispositif de précontrainte (48) pousse l'électrode (16) en direction d'une position (A) complètement déployée.

10. Sonde selon la revendication 8 ou 9, **caractérisée en ce que** le dispositif de précontrainte (48) est installé dans le corps de sonde (12) et/ou dans le boîtier (21).

11. Sonde selon une des revendications précédentes, **caractérisée en ce que** le dispositif de commande (20) présente un élément de commande de sonde à jet d'eau (66) qui est couplé en mouvement au corps de sonde à jet d'eau (60) et est agencé pour déplacer le corps de sonde à jet d'eau (60) dans le sens de son étendue (R) par rapport au boîtier (21) du dispositif de commande (20).

12. Sonde selon la revendication 11, **caractérisée en ce que** dans la première position (I), l'élément de commande d'électrode (23) constitue une butée pour l'élément de commande de sonde à jet d'eau (66).

13. Sonde selon une des revendications 1 à 10, **caractérisée en ce que** l'élément de commande d'électrode (23) est conçu aussi bien pour le déplacement de l'électrode (16) que pour le déplacement du corps de sonde à jet d'eau (60).

14. Sonde selon la revendication 13, **caractérisée en ce que** l'élément de commande d'électrode (23) est conçu pour provoquer d'abord un mouvement d'escamotage de l'électrode (16), lors d'un déplacement depuis la première position (I) en direction de la deuxième position (II), et pour provoquer ensuite un mouvement de déploiement du corps de sonde à jet d'eau (60).

15. Sonde selon une des revendications précédentes, **caractérisée en ce qu'**il est prévu dans le boîtier (21), un dispositif de déviation (69) qui est conçu pour coupler le corps de sonde à jet d'eau (60) à un élément de commande (23, 66) associé du dispositif de commande (20), de manière à ce que les sens de déplacement du corps de sonde à jet d'eau (60) installé à l'extérieur du boîtier (21) et de l'élément de commande (23, 66) soient opposés l'un à l'autre.
